# EUROPÄISCHE PATENTSCHRIFT

(11) **EP 3 166 471 B1**
(45) Veröffentlichungstag und Bekanntmachung des Hinweises auf die Patenterteilung: **20.12.2017**
(21) Anmeldenummer: 15777628.7
(22) Anmeldetag: 22.09.2015
(51) Int. Cl.: A61B 3/00, A61B 5/00, A61B 3/032, A61B 3/04, A61B 3/06, A61B 3/10, A61B 3/11, A61B 3/14, G02C 7/02

(54) **VERFAHREN UND VORRICHTUNGEN ZUR BESTIMMUNG DER AUGENREFRAKTION**
METHOD AND DEVICES FOR TESTING EYE REFRACTION
PROCÉDÉ ET DISPOSITIFS DE DÉTERMINATION DE RÉFRACTION OCULAIRE

(30) Priorität: 22.09.2014 DE 102014113680; 14.11.2014 DE 102014116665
(43) Veröffentlichungstag der Anmeldung: 17.05.2017
(60) Teilanmeldung: 17155794.5
(73) Patentinhaber: Carl Zeiss AG, 73447 Oberkochen (DE); Carl Zeiss Vision International GmbH, 73430 Aalen (DE)
(72) Erfinder: LINDIG, Karsten, 99084 Erfurt (DE); CABEZA-GUILLÉN, Jesús-Miguel, 73434 Aalen (DE)
(74) Vertreter: Sticht, Andreas
(86) Internationale Anmeldenummer: PCT/EP2015/071690
(87) Internationale Veröffentlichungsnummer: WO 2016/046186

(56) Entgegenhaltungen:
- WO-A1-2013/170091
- WO-A2-2013/036629
- DE-A1- 4 326 760
- DE-A1- 10 348 854
- US-A1- 2007 200 927
- US-A1- 2009 153 796
- US-A1- 2011 157 550
- US-A1- 2012 274 902
- US-A1- 2013 027 668
- US-A1- 2013 083 185
- US-A1- 2014 211 167
- US-A1- 2014 268 060

## Beschreibung

Die vorliegende Anmeldung betrifft Verfahren, Vorrichtungen und Systeme zum Bestimmen der Augenrefraktion und entsprechende Computerprogrammprodukte. Unter einer Augenrefraktion ist dabei eine Bestimmung einer Refraktion eines oder beider Augen eines Lebewesens, insbesondere eines Menschen zu verstehen. Dabei werden üblicherweise Werte für Sphäre (in Dioptrien), Zylinder und Achse bestimmt. Eine derartige Bestimmung der Augenrefraktion wird beispielsweise benutzt, um Sehhilfen wie Brillen oder Kontaktlinsen an eine jeweilige Person anzupassen.

Im medizinischen Bereich sind herkömmliche Diagnosesysteme zur Bestimmung der Augenrefraktion in der Regel als einzelne Table-Top-Systeme ausgeführt. Zur objektiven Bestimmung der Augenrefraktion wird dabei beispielsweise ein Autorefraktor oder ein Oszilloskop verwendet. Neben der objektiven Refraktionsbestimmung mittels derartiger Geräte, bei denen eine objektive Messung der Refraktion des Auges erfolgt, werden häufig auch sogenannte subjektive Refraktionsbestimmungen durchgeführt, bei welchem einer Person verschiedene Linsen oder andere optische Elemente mit verschiedenen optischen Eigenschaften bereitgestellt werden (beispielsweise in einem Brillengestell mit einer Halterung für derartige Linsen) und die Person angibt, ob sich ein Seheindruck verbessert oder verschlechtert. Hierfür betrachtet die Person üblicherweise eine Sehtafel mit Zeichen oder Symbolen, welche sich in größerer Entfernung, beispielsweise 4 m, befindet.

Somit wird herkömmlicherweise für die Bestimmung der Augenrefraktion relativ viel Platz benötigt. Zudem sind häufig hierfür nötige Vorrichtungen relativ teuer. Daher ist es wünschenswert, kompaktere Vorrichtungen zur Bestimmung der Augenrefraktion bereitzustellen.

Aus der US 2013/0235346 A1 ist es bekannt, ein Smartphone oder andere Computereinrichtungen zur Augenuntersuchung, insbesondere zur Photorefraktion, zu verwenden. Dabei wird mittels einer Kamera des Smartphones ein Auge oder werden beide Augen einer zu untersuchenden Person aufgenommen, und eine Beleuchtung wird beispielsweise über einen Blitz oder andere Leuchteinrichtung des Smartphones bereitgestellt.

Aus der US 2012/0212598 A1 ist es bekannt, ein zu untersuchendes Auge mittels einer Leuchtdiodenanordnung zu beleuchten, um ein Auge aus verschiedenen Richtungen beleuchten zu können. Es wird hier also wiederum ein spezialisiertes Gerät bereitgestellt, welches unter Umständen vergleichsweise teuer ist.

Aus der DE 10153397 A1 sind weitere Verfahren und Vorrichtungen zur Messung der Refraktion eines Auges bekannt, bei welchen ein Auge beleuchtet wird und dann ein Bild des Auges ausgewertet wird.

Die US 2013/0027668 A1 offenbart ein Verfahren zur subjektiven Refraktionsbestimmung, wofür eine mobile Anzeigevorrichtung, insbesondere ein Smartphone, verwendet werden kann. Dabei werden Bilder angezeigt, die von einem Probanden zur Deckung gebracht werden müssen.

Aus der DE 10 2008 012 268 A1 ist eine stationäre Vorrichtung zur objektiven Refraktionsbestimmung bekannt.

Die US 2006/0110008A1 offenbart Verfahren und Vorrichtungen zur kalibrierungsfreien Augennachverfolgung ("eye gaze tracking"),

Die DE 20 2011 103 183 U1 offenbart einen Bildtrenner, welcher zur Bestimmung von Defiziten der Sehschärfe und Refraktion eines Auges an einem Tablet-Rechner angebracht werden kann.

Die US 2009/0153796 A1 offenbart eine Vorrichtung mit einer am Kopf anzubringenden Einheit und einer zentralen Recheneinheit. Die Recheneinheit steuert die am Kopf getragene Einheit an, Testmuster darzustellen, um eine Refraktion von Linsen an einen Benutzer anzupassen.

Die WO 2013/170091 A1 offenbart Verfahren und Vorrichtungen zur Messung der visuellen Empfindlichkeit, wobei die Bestimmung unter verschiedenen Beleuchtungsbedingungen erfolgen kann.

Die US 2011/0157550 A1 offenbart, Leuchtdiodenmuster zu erzeugen, welche zur Bestimmung von Retinaeigenschaften oder Refraktionseigenschaften dienen.

Die US 2013/0027668 A1 offenbart eine Vorrichtung zur subjektiven Refraktionsbestimmung, bei welcher eine Helligkeit einstellbar ist, um ein Hintergrundbild zu beleuchten.

Die US 2013/0083185 A1 offenbart ein optisches Adaptersystem für eine ophthalmologische bildgebende Vorrichtung.

Die DE 103 48 854 A1 offenbart ein Verfahren und eine Vorrichtung zur Ermittlung der Restfehlsichtigkeit eines Patienten, bei welcher ein Strahlteiler zwischen einem Auge einer zu untersuchenden Person und einer Sehtafel angeordnet ist, wobei der Strahlteiler Licht von dem Auge zu einem Kamerachip lenkt. Polarisatoren dienen dabei zur Infrarotbeleuchtung und zur Unterdrückung von Linsenreflexionen.

Die US 2012/0274902 A1 offenbart eine tabletförmige Messeinrichtung zur Augenvermessung, welche mit einem Computer kommuniziert. Zur Beleuchtung können verschiedene Leuchtdioden aktiviert werden, sodass ein Pupillenreflex entsprechend wandert.

Die DE 432 760 A1 offenbart eine optische Anordnung zum Betrachten eines Displays, welche zur Prüfung zentraler Sehfunktionen verwendet werden kann.

Die US 2007/0200927 A1 offenbart es, die Sehschärfe mittels einer am Kopf zu tragenden Vorrichtung als Funktion der Helligkeit zu messen.

Ausgehend hiervon ist es eine Aufgabe der vorliegenden Erfindung, Vorrichtungen, Systeme und Computerprogrammprodukte zur Bestimmung der Augenrefraktion bereitzustellen, welche beispielsweise verglichen mit herkömmlicheren Herangehensweisen kostengünstiger zu implementieren sind und/oder eine erhöhte Genauigkeit bieten und/oder flexibler einsetzbar sind als herkömmliche stationäre Einrichtungen.

Es werden eine mobile Computervorrichtung nach Anspruch 1, ein System mit einer derartigen Computervorrichtung nach Anspruch 9, ein Verfahren nach Anspruch 10 sowie ein Computerprogrammprodukt nach Anspruch 15 bereitgestellt. Die Unteransprüche definieren weitere Ausführungsbeispiele.

Gemäß einem ersten Aspekt wird eine mobile Computervorrichtung bereitgestellt, umfassend eine Anzeige, einen Prozessor, und einen Speicher mit darin gespeichertem Programmcode, wobei der Programmcode, wenn er auf dem Prozessor ausgeführt wird, bewirkt, dass der Prozessor die Anzeige ansteuert, um auf der Anzeige ein Bild (d.h. ein Bild oder mehrere aufeinanderfolgende Bilder) zur Refraktionsbestimmung eines Auges anzuzeigen.

Dabei umfasst das Bild auf einem Hintergrund dargestellte Elemente, wobei eine Helligkeit des Hintergrundes zur Einstellung einer Umfeldleuchtdichte, insbesondere einer Adaptionsleuchtdichte, einstellbar ist. Der erste Aspekt ist dadurch gekennzeichnet, dass die Helligkeit des Hintergrundes wahlweise auf einen von mindestens zwei unterschiedlichen vorgegebenen Helligkeitswerten einstellbar ist, wobei die mindestens zwei unterschiedlichen vorgegebenen Helligkeitswerte aus einer Gruppe sind bestehen aus einem ersten vorgegebenen Helligkeitswert mit einer ersten Leuchtdichte kleiner als 0,03 cd/m2, einem zweiten vorgegebenen Helligkeitswert mit einer zweiten Leuchtdichte größer als 3 cd/m2 und einem dritten Helligkeitswert mit einer dritten Leuchtdichte zwischen der ersten Leuchtdichte und der zweiten Leuchtdichte.

Die erste Leuchtdichte liegt dabei im Bereich skotopischen Sehens, die zweite Leuchtdichte im Bereich des photopischen Sehens und die dritte Leuchtdichte im Bereich des mesopischen Sehens.

Photopisches Sehen, auch Tagsehen oder Zapfensehen, bezeichnet das Sehen des Menschen bei ausreichender Helligkeit. Im Gegensatz dazu steht das skotopische Sehen, auch Nachtsehen oder Stäbchensehen, bei geringer Helligkeit und dem Übergangsbereich, dem mesopischen Bereich oder Dämmerungssehen. Skotopisches Sehen findet typischerweise von der Wahrnehmungsgrenze bis zu einer Leuchtdichte von etwa 0,003-0,03 cd/m2 statt. Der Bereich des mesopischen Sehens liegt darüber bis zu Leuchtdichten von etwa 3 - 30 cd/m2. Bei noch höheren Leuchtdichten findet photopisches Sehen statt. Gemäß dem ersten Aspekt kann also die Helligkeit des Hintergrundes wahlweise auf mindestens zwei dieser drei Bereiche eingestellt werden, um eine Refraktionsbestimmung in dem entsprechenden Sehbereich (photopisch, skotopisch oder mesopisch) durchzuführen. Auf diese Weise kann mit einer einzigen Vorrichtung die Refraktionsbestimmung in verschiedenen Bereichen des Sehens durchgeführt werden.

Zudem kann eine Helligkeit der Elemente zur Einstellung einer Infeldleuchtdichte einstellbar sein.

Die Infeldleuchtdichte ist dabei wie in der Optik üblich die durchschnittliche Leuchtdichte aller im Infeld befindlichen Objekte. Das Infeld ist dabei allgemein die Fläche eines betrachteten Sehobjektes als Position innerhalb des gesamten Gesichtsfeldes. Das Infeld befindet sich für ein derartiges Sehobjekt im Allgemeinen in der Hauptblickrichtung des entsprechenden Auges und wird in seiner Größe durch die Größe des Sehobjektes bei geringen Blickbewegungen bestimmt. Es ist im Allgemeinen nicht größer als 20° und wird vom Umfeld umgeben. Die Umfeldleuchtdichte ist dann entsprechend die durchschnittliche Leuchtdichte des Umfeldes.

Dies gibt eine hohe Flexibilität bei der Einstellung eines Kontrastes zwischen Elementen und Hintergrund sowie bei einer Einstellung einer Adaptionsleuchtdichte, die die Augenadaption bestimmt.

Das Bild kann bei dem ersten Aspekt ein Bild zur subjektiven Refraktionsbestimmung umfassen, wobei das Bild beispielsweise Zeichen und/oder Symbole umfasst.

Das Bild kann auch eine strukturierte Beleuchtung zur Beleuchtung des Auges für eine objektive Refraktionsbestimmung umfassen. Eine strukturierte Beleuchtung ist dabei eine Beleuchtung, die im Gegensatz zu eine gleichförmigen Beleuchtung eine räumliche Struktur aufweist, insbesondere ein Muster (was einer räumlichen Änderung der Helligkeit und/oder der Farbe entspricht) oder eine andere Art der räumlichen Modulation.

Die strukturierte Beleuchtung kann zeitlich veränderlich sein, um insbesondere das Auge aus verschiedenen Richtungen zu beleuchten. Beispielsweise können hierzu nacheinander verschiedene Teile eines Musters auf "hell" geschaltet werden, während andere Teile des Musters dunkel bleiben. Hierdurch kann das Auge mit Beleuchtung aus verschiedenen Richtungen untersucht werden.

Die strukturierte Beleuchtung kann dabei wahlweise ansteuerbare kreisförmig angeordnete Lichtquellenpunkte oder Ringsegmente umfassen.

Die mobile Computervorrichtung kann weiter eine Kameraeinrichtung zum Aufnehmen eines Pupillenreflexes in Antwort auf die strukturierte Beleuchtung umfassen.

Zusätzlich oder alternativ kann die mobile Computervorrichtung eine Schnittstelle zum Koppeln mit einer externen Kamera und zum Empfangen eines aufgenommenen Pupillenreflexes in Antwort auf die strukturierte Beleuchtung von der externen Kamera umfassen.

Der Programmcode, wenn er auf dem Prozessor ausgeführt wird, kann dann eine Refraktionsbestimmung des Auges auf Basis des aufgenommenen Pupillenreflexes durchführen.

Gemäß einem zweiten Aspekt wird ein System bereitgestellt, umfassend eine mobile Computervorrichtung wie oben beschrieben, und eine Betrachtungsoptik zum Betrachten der mobilen Computervorrichtung mit mindestens einem Auge einer zu untersuchenden Person.

Die Betrachtungsoptik umfasst eine erste Optik für ein erstes Auge und eine zweite Optik für ein zweites Auge, um eine binokulare Betrachtung und/oder Untersuchung zu ermöglichen

Zusätzlich kann die Betrachtungsoptik eine Mikrolinsenanordnung umfassen. Eine Mikrolinsenanordnung ist dabei eine Anordnung einer Vielzahl von Mikrolinsen (d.h. kleiner Linsen, welche beispielsweise in einer zweidimensionalen Anordnung in Zeilen und Spalten bereitgestellt sein können.

Die Mikrolinsenanordnung kann insbesondere eine auf der Anzeige der mobilen Computervorrichtung anzubringende Mikrolinsenfolie umfassen. Damit ist ein kompakter Aufbau möglich.

Die Betrachtungsoptik kann auch einen Farbfilter umfassen. Dabei kann der Farbfilter einen Rotfilter und/oder Infrarotfilter umfassen. Hierdurch kann erreicht werden, dass von dem angezeigten Bild nur Licht der durch den Filter ausgewählten Wellenlängen das Auge erreicht. Insbesondere Rotlicht oder Infrarotlicht hat dabei einen geringeren Einfluss auf die Adaption der Pupille, so dass z.B. mit vergleichsweise hoher Lichtintensität und trotzdem vergleichsweise großer Pupille gearbeitet werden kann.

Das System kann weiter einen Strahlteiler umfassen, welcher zwischen der Anzeige der mobilen Computervorrichtung und dem mindestens einen Auge angeordnet ist, wobei der Strahlteiler angeordnet ist, von dem Auge ausgehendes Licht zu einer Kameraeinrichtung zu lenken.

Das System kann zudem eine Wechselhalterung für eine Wechseloptik zur subjektiven Refraktionsbestimmung umfassen.

Das System kann weiter, für mindestens ein Auge, eine Kombination aus Polarisator und Analysator umfassen. Hierdurch kann für das mindestens eine Auge Fremdlicht, z.B. aus einem Kanal für ein anderes Auge, unterdrückt werden.

Das System kann weiter, für mindestens ein Auge, einen Shutter umfassen.

Gemäß einem dritten Aspekt wird ein Verfahren zur Augenuntersuchung bereitgestellt, umfassend:
Anzeigen eines Bildes zur Refraktionsbestimmung eines Auges auf einer Anzeige (11) einer mobilen Computervorrichtung, wobei das Bild auf einem Hintergrund dargestellte Elemente umfasst, und
Einstellen einer Helligkeit des Hintergrundes zur Einstellung einer Umfeldleuchtdichte
   wobei die Helligkeit des Hintergrundes wahlweise zur Refraktionsbestimmung für mindestens zwei Arten von Sehen aus der Gruppe bestehend aus skotopisches Sehen, photopisches Sehen und mesopisches Sehen eingestellt wird.

Wie bei dem ersten Aspekt ist so auf einfache Weise eine Refraktionsbestimmung für verschiedene Sehbereiche (skotopisch, photopisch, mesopisch) möglich.

Bei dem Verfahren des dritten Aspekts weist die Helligkeit des Hintergrundes für das skotopische Sehen eine Leuchtdichte kleiner als 0,03 cd/m2 auf und die Helligkeit des Hintergrundes für das photopische Sehen eine Leuchtdichte größer als 3 cd/m2 auf.

Ähnlich dem ersten Aspekt umfasst bei dem dritten Aspekt das Einstellen der Helligkeit des Hintergrundes ein wahlweises Einstellen der Helligkeit auf einen von mindestens zwei unterschiedlichen vorgegebenen Helligkeitswerten, wobei die mindestens zwei unterschiedlichen vorgegebenen Helligkeitswerte aus einer Gruppe sind bestehen aus einem ersten vorgegebenen Helligkeitswert mit einer ersten Leuchtdichte kleiner als 0,03 cd/m2, einem zweiten vorgegebenen Helligkeitswert mit einer zweiten Leuchtdichte größer als 3 cd/m2 und einem dritten Helligkeitswert mit einer dritten Leuchtdichte zwischen der ersten Leuchtdichte und der zweiten Leuchtdichte.

Das Verfahren kann weiter umfassen:
Einstellen einer Helligkeit der Elemente zur Einstellung einer Infeldleuchtdichte.

Die Begriffe Infeldleuchtdichte und Umfeldleuchtdichte sind dabei wie in der Optik gebräuchlich und wie bei dem ersten erläutert zu verstehen.

Bei dem Verfahren kann das Bild eine strukturierte Beleuchtung zur Beleuchtung des Auges für eine objektive Refraktionsbestimmung umfassen.

Eine strukturierte Beleuchtung ist dabei eine Beleuchtung, die im Gegensatz zu eine gleichförmigen Beleuchtung eine räumliche Struktur aufweist, insbesondere ein Muster (was einer räumlichen Änderung der Helligkeit und/oder der Farbe entspricht) oder eine andere Art der räumlichen Modulation.

Das Verfahren kann dann weiter umfassen:
zeitliches Verändern der strukturierten Beleuchtung, um das Auge aus verschiedenen Richtungen zu beleuchten.

Beispielsweise können hierzu nacheinander verschiedene Teile eines Musters auf "hell" geschaltet werden, während andere Teile des Musters dunkel bleiben. Hierdurch kann das Auge mit Beleuchtung aus verschiedenen Richtungen untersucht werden.

Das Verfahren kann weiter ein Aufnehmen eines Pupillenreflexes in Antwort auf die strukturierte Beleuchtung umfassen. Der Pupillenreflex kann Hinweise auf Eigenschaften des Auges wie Blickrichtung oder Refraktion geben.

Das Verfahren kann weiter ein Durchführen einer Refraktionsbestimmung des Auges auf Basis des aufgenommenen Pupillenreflexes mittels eines auf einem Prozessor der mobilen Computervorrichtung ausgeführten Programmcodes umfassen. Die Refraktionsbestimmung kann dabei auf Basis einer Änderung einer Position des Reflexes bei Änderung der strukturierten Beleuchtung erfolgen, wie später noch näher erläutert werden wird.

Gemäß einem vierten Aspekt wird ein Computerprogrammprodukt bereitgestellt, welches in einen Speicher einer mobilen Computervorrichtung ladbar ist, umfassend Softwarecode, welcher eines der oben beschriebenen Verfahren ausführt, wenn er auf der mobilen Computervorrichtung ausgeführt wird. Das Computerprogrammprodukt kann beispielsweise ein physikalisches Medium sein, auf dem der Softwarecode bereitgestellt wird, beispielsweise eine Speicherkarte, ein USB-Stick, eine DVD-ROM, eine CD-ROM oder eine andere Art von Speicher. Es kann sich aber auch um Signale oder dergleichen handeln, mit denen der Softwarecode bereitgestellt wird.

Somit ist erfindungsgemäß mittels einer mobilen Computervorrichtung, insbesondere einem Smartphone oder einem Tablet, sowohl eine objektive Refraktionsmessung als auch eine subjektive Refraktionsmessung möglich.

Dabei können die oben beschriebenen Techniken, Vorrichtungen und Verfahren zur objektiven Refraktionsmessung und zur subjektiven Refraktionsmessung in einer einzigen Vorrichtung kombiniert sein, es können jedoch ebenso auch getrennte Vorrichtungen bereitgestellt sein.

Ausführungsbeispiele der vorliegenden Erfindung werden nachfolgend unter Bezugnahme auf die beigefügten Zeichnungen näher erläutert. Es zeigen:
Fig. 1 ein Blockdiagramm einer mobilen Computervorrichtung gemäß einem Ausführungsbeispiel,
Fig. 2 eine schematische Außenansicht einer mobilen Computervorrichtung gemäß einem Ausführungsbeispiel,
Fig. 3 ein Flussdiagramm zur Veranschaulichung der Funktionsweise von mobilen Computervorrichtungen gemäß Ausführungsbeispielen,
Fig. 4 eine Darstellung einer möglichen strukturierten Beleuchtung zur Durchführung einer objektiven Refraktionsbestimmung gemäß einem Ausführungsbeispiel,
Fig. 5 eine Alternative zu der strukturierten Beleuchtung der Fig. 5,
Fig. 6 bis 12 verschiedene Varianten von Systemen zur objektiven Refraktionsbestimmung gemäß verschiedenen Ausführungsbeispielen,
Fig. 13 ein Blockdiagramm zur Veranschaulichung der Funktionsweise von Systemen zur objektiven Refraktionsbestimmung gemäß einem Ausführungsbeispiel,
Fig. 14 bis 16 Systeme zur subjektiven Refraktionsbestimmung gemäß verschiedenen Ausführungsbeispielen, und
Fig. 17A bis 17D Beispiele für Bildschirmdarstellungen zur subjektiven Refraktionsbestimmung gemäß Ausführungsbeispielen.

Im Folgenden werden verschiedene Ausführungsbeispiele der vorliegenden Erfindung unter Bezugnahme auf die beigefügten Zeichnungen detailliert erläutert. Diese Ausführungsbeispiele dienen lediglich der Veranschaulichung und sind nicht als einschränkend auszulegen. Beispielsweise bedeutet eine Beschreibung eines Ausführungsbeispiels mit einer Vielzahl von Elementen oder Merkmalen nicht, dass alle diese Elemente oder Merkmale zur Implementierung von Ausführungsbeispielen notwendig sind. Vielmehr können andere Ausführungsbeispiele weniger Merkmale oder Elemente, alternative Merkmale oder Elemente und/oder zusätzliche Merkmale oder Elemente aufweisen. Zudem können Merkmale oder Elemente verschiedener Ausführungsbeispiele miteinander kombiniert werden, sofern nichts anderes angegeben ist.

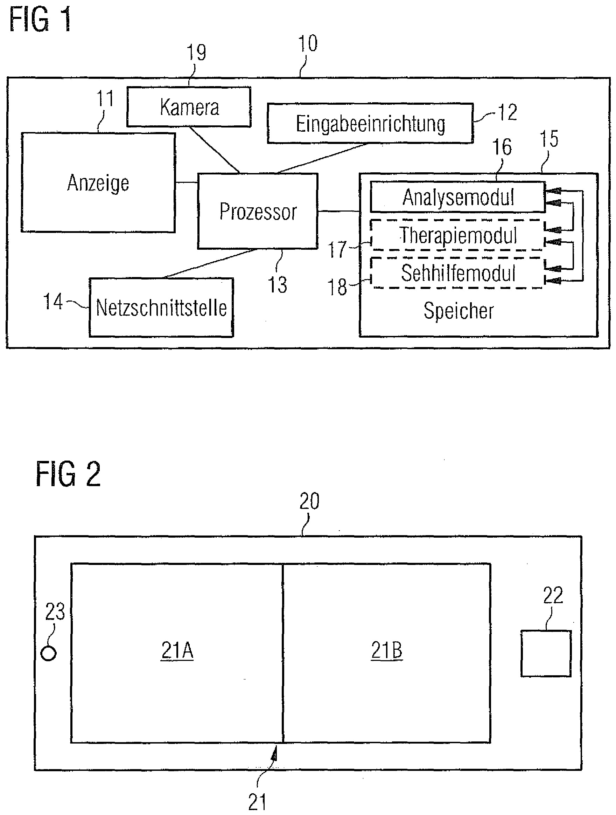

In Fig. 1 ist eine mobile Computervorrichtung 10 gemäß einem Ausführungsbeispiel dargestellt. Die mobile Computervorrichtung 10 kann beispielsweise mittels eines Smartphones, eines Tablet-Computers oder mittels einer anderen mobilen Computervorrichtung (beispielsweise einem mobilen Spielgerät) implementiert sein. Derartige mobile Computervorrichtungen, welche als Grundlage für die Implementierung der mobilen Computervorrichtung 10 der Fig. 1 dienen können, sind häufig frei programmierbar, verfügen über einen Prozessor, eine Anzeige (Display, gegebenenfalls berührungsempfindlich), verschiedene Eingabeeinrichtungen, Netzschnittstellen etc. Wie im Folgenden näher erläutert wird, werden bei Ausführungsbeispielen der vorliegenden Erfindung derartige mobile Computervorrichtungen genutzt, um Möglichkeiten zur Refraktionsbestimmung bereitzustellen. Hierzu kann insbesondere die mobile Computervorrichtung entsprechend programmiert werden, beispielsweise mittels einer oder mehrerer sogenannter Apps (vom Englischen "applications", d.h. Anwenderprogramme).

Als Beispiel umfasst die mobile Computervorrichtung der Fig. 1 einen Prozessor 13, beispielsweise eine CPU. Bei anderen Ausführungsbeispielen können auch mehrere Prozessoren oder ein Prozessor mit mehreren Prozessorkernen bereitgestellt sein. Der Prozessor 13 ist mit einem Speicher 15, beispielsweise einem Speicher mit wahlfreiem Zugriff (RAM) oder einem nicht-flüchtigen Speicher wie einem Flashspeicher gekoppelt, oder Kombinationen hiervon. In dem Speicher 15 können Daten sowie Programme zum Betreiben des Prozessors 13 und der mobilen Computervorrichtung 10 abgelegt sein. Insbesondere können verschiedene Anwendungsprogramme (Apps) in dem Speicher 15 abgelegt sein, beispielsweise Anwendungsprogrammmodule 16 und 17, welche später näher diskutiert werden.

Der Prozessor 13 ist weiter mit einer Anzeige 11 gekoppelt, über welche Bilder, z.B. Informationen, Grafiken, Zeichen, Symbole und dergleichen zum Betrachten durch einen Benutzer dargestellt werden können. Der Bergriff "Bild" wird hier allgemein benutzt, um Inhalte zu bezeichnen, welche auf einer Anzeige dargestellt werden. Ein derartiges Bild kann beispielsweise Zeichen, Symbole und/oder andere Elemente umfassen. Des Weiteren ist der Prozessor 13 mit einer Eingabeeinrichtung 12 gekoppelt. Bei manchen Ausführungsbeispielen kann die Anzeige 11 berührungsempfindlich sein und somit gleichzeitig die Eingabeeinrichtung 12 oder einen Teil hievon darstellen. Zusätzlich oder alternativ kann die Eingabeeinrichtung Knöpfe, Drehregler, eine Tastatur, ein Mikrofon zum Empfangen von Geräuschen oder einer Spracheingabe und/oder Sensoren wie einen Neigungssensor oder einen Beschleunigungssensor umfassen.

Des Weiteren weist bei dem Ausführungsbeispiel der Fig. 1 die mobile Computervorrichtung 10 eine oder mehrere Kameras 19 auf. Beispielsweise weisen moderne Smartphones typischerweise zumindest eine Frontkamera, welche sich auf der gleichen Seite wie die Anzeige 11 befindet, sowie eine rückseitige Kamera auf der gegenüberliegenden Seite auf, wobei in vielen Fällen die rückseitige Kamera eine höhere Auflösung bietet. Es sind jedoch auch andere Konfigurationen möglich. Bei manchen Ausführungsbeispielen wird eine derartige Kamera, wie die Kamera 19, verwendet, ein Bild eines oder beider Augen eines Benutzers aufzunehmen, um auf Basis hierauf eine objektive Refraktionsbestimmung durchzuführen. Bei anderen Ausführungsbeispielen kann hierfür eine externe Kamera dienen.

Des Weiteren umfasst die mobile Computervorrichtung 10 der Fig. 1 eine Netzschnittstelle 14, mittels der die Computervorrichtung 10 mit weiteren Vorrichtungen kommunizieren kann. Die Netzschnittstelle 14 kann beispielsweise eine Mobilfunkschnittstelle zur Kommunikation über ein Mobilfunknetz, eine Bluetooth-Schnittstelle und/oder eine Wifi/WLAN-Schnittstelle umfassen, ist jedoch nicht hierauf beschränkt. Eine derartige Netzschnittstelle 14 kann beispielsweise zur Kommunikation mit einer oder mehreren externen Kamera(s) dienen, beispielsweise über Bluetooth. Eine derartige externe Kamera kann, wie später näher erläutert werden wird, ebenfalls zur Aufnahme eines oder beider Augen eines Benutzers dienen. Zu bemerken ist, dass die mobile Computervorrichtung 10 weitere herkömmliche Komponenten von mobilen Computervorrichtungen wie beispielsweise Smartphones aufweisen kann.

Bei dem dargestellten Ausführungsbeispiel sind in dem Speicher 15 als Anwendungsprogramme ein Analysemodul 16 zur Refraktionsbestimmung und optional zusätzlich ein weiteres Modul 17 abgelegt.

Wie durch Pfeile angedeutet können die Module auch miteinander interagieren, insbesondere Daten austauschen. Das Analysemodul 16 dient dazu, mittels der mobilen Computervorrichtung eine Refraktionsbestimmung durchzuführen. Beispielsweise können zur objektiven Refraktionsbestimmung mit einer internen Kamera wie der Kamera 19 der Fig. 1 oder einer mit der mobilen Computervorrichtung gekoppelten externen Kamera Bilder eines oder beider Augen eines Benutzers aufgenommen werden, und die Bilder können ausgewertet werden. Zusätzlich kann hierzu die Anzeige wie die Anzeige 11 angesteuert werden, ein oder mehrere Bilder, welche eine Beleuchtung für das Auge oder die Augen, insbesondere eine strukturierte Beleuchtung bilden, darzustellen. Eine strukturierte Beleuchtung kann dabei insbesondere eine Beleuchtung mit einem vorgegeben, gegebenenfalls veränderlichen, Muster sein.

Zudem kann die Anzeige 11 angesteuert werden, um Bilder, z.B. Symbole, beispielsweise Zeichen oder Zahlen, zur Durchführung einer subjektiven Refraktionsbestimmung anzuzeigen. Dies kann bei manchen Ausführungsbeispielen mit variabler Hintergrundhelligkeit erfolgen.

Zudem kann optional ein weiteres Modul 17 bereitgestellt sein, welches mit dem Analysemodul Daten austauschen kann. Das weitere Modul 17 kann beispielsweise Bestellfunktionen für Sehhilfen auf Basis der bestimmten Augenrefraktion oder gegebenenfalls auch Therapie-und/oder Sehhilfefunktionen bereitstellen.

Zudem ist über das weitere Modul 17 beispielsweise auch eine Kommunikation der Ergebnisse über die Netzschnittstelle 14 beispielsweise zu einem Augenarzt oder anderem medizinischen Fachpersonal möglich.

Es ist zu bemerken, dass das Analysemodul 16 bei manchen Ausführungsbeispielen auch nur Teilaufgaben der Refraktionsbestimmung übernehmen kann. Beispielsweise können auch Rohdaten an eine weitere Computervorrichtung gesendet werden, und die aufgenommenen Daten (beispielsweise Bilder) können dort ausgewertet werden.

Mit einer derartigen mobilen Computervorrichtung sind bei Ausführungsbeispielen und Messungen bei kontrollierten lichttechnischen Verhältnissen möglich. Damit lassen sich bei verschiedenen Adaptionspegeln und damit verschiedenen Pupillengrößen Refraktionsbestimmungen durchführen. Zudem weisen viele Anzeigen heutiger Smartphones oder Tablets einen hohen Dynamikbereich auf. Dies ermöglicht Beleuchtungen zur Analyse mit sehr hohem Kontrastumfang. Beispielsweise weisen Anzeigen auf Basis organischer Leuchtdioden (OLED) einen Kontrastumfang von 1000:1 oder mehr auf, was für die Bereitstellung einer Beleuchtung für das Auge hilfreich sein kann.

In Fig. 2 ist ein Beispiel für eine Außenansicht einer mobilen Vorrichtung 20 gemäß einem Ausführungsbeispiel der vorliegenden Erfindung dargestellt. Die mobile Vorrichtung 20 der Fig. 2 kann beispielsweise der mobilen Computervorrichtung 10 der Fig. 1 entsprechen und ist in dem Beispiel der Fig. 2 als Smartphone ausgestaltet. Je nach Art des Smartphones kann sich die Außenansicht von der in Fig. 2 dargestellten unterscheiden. Die mobile Computervorrichtung 20 weist eine Anzeige 21 auf, welche insbesondere als berührungsempfindliche Anzeige (sogenannter Touchscreen) realisiert sein kann. Zudem weist bei dem dargestellten Beispiel die mobile Computervorrichtung 20 einen Bedienknopf 22 sowie eine Kamera 23 auf. Die Kamera 23 wird auch als Frontkamera bezeichnet. Eine weitere (in Fig. 2 nicht dargestellte) Kamera kann sich auf der gegenüberliegenden Seite (Rückseite) der mobilen Computervorrichtung 20 befinden. Die mobile Computervorrichtung 20 kann weitere in Fig. 2 nicht dargestellte Elemente aufweisen, wie beispielsweise einen Kopfhörerausgang, weitere Bedienelemente, einen Lautsprecher oder ein Mikrofon.

Wie in Fig. 2 dargestellt kann die Anzeige 21 in zwei Bereiche 21A und 21B unterteilt werden. In diesem Fall kann beispielsweise der Bereich 21A zur Darstellung eines Bildes und/oder einer Beleuchtung für ein linkes Auge eines Benutzers und der Bereich 21B zur Darstellung eines Bildes und/oder Beleuchtung für ein rechtes Auge eines Benutzers genutzt werden. Wie nachfolgend näher erläutert werden wird, kann die mobile Computervorrichtung 20 in eine entsprechende Betrachtungsvorrichtung eingesetzt werden.

Durch die Aufteilung der Anzeige wie in Fig. 2 dargestellt können beispielsweise durch das Analysemodul 16 linkes und rechtes Auge getrennt getestet werden, beispielsweise indem nur in dem Bereich 21A oder nur in dem Bereich 21B etwas angezeigt wird und eine entsprechende Rückmeldung von einem Benutzer ausgewertet wird, oder es können Stereosehfähigkeiten und dergleichen getestet werden.

In den Bereichen 21A und 21B können beispielsweise Bilder für linkes und rechtes Augen entsprechend einem Pupillenabstand dargestellt werden, um eine Stereobetrachtung zu ermöglichen. Beispielsweise kann ein Abstand korrespondierender Punkte so angepasst sein, dass er in etwa dem Pupillenabstand eines erwachsenen Menschen entspricht, beispielsweise ungefähr 65 mm. Bei manchen Ausführungsbeispielen kann der Pupillenabstand auch ein Parameter sein, sodass die Darstellung an einem jeweiligen Benutzer und dessen Pupillenabstand angepasst werden kann.

Mobile Computervorrichtungen wie in Fig. 1 und 2 dargestellt sind häufig vergleichsweise kostengünstig, da sie in großen Stückzahlen hergestellt werden. Dennoch sind in derartigen Vorrichtungen häufig hochwertige Anzeigen (Displays) Kameras, Sensoren etc. eingesetzt.

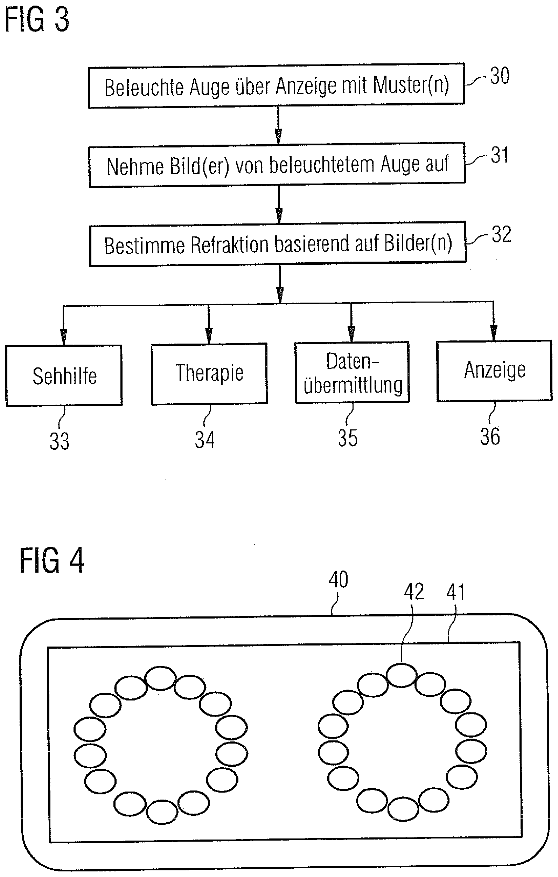

In Fig. 3 ist ein Flussdiagramm zur Veranschaulichung eines Verfahrens zur objektiven Refraktionsbestimmung dargestellt. Das Verfahren kann beispielsweise durch das Analysemodul 16 die Fig. 1 mittels einer mobilen Computervorrichtung wie der mobilen Computervorrichtung 10 der Fig. 1 implementiert sein.

Zur Vereinfachung wird dabei bei der Beschreibung der Fig. 3 nur ein Auge erwähnt. Die verschiedenen Schritte der Fig. 3 sind jedoch auf beide Augen, hintereinander oder simultan, anwendbar. "Ein" bei "ein Auge" ist also als unbestimmter Artikel und nicht als einschränkende Zahlenangabe zu verstehen.

In einem Schritt 30 wird ein Auge eines Benutzers über eine Anzeige einer mobilen Computervorrichtung (beispielsweise Anzeige 11 der Fig. 1 oder Anzeige 21 der Fig. 2) mit einem Muster, welches sich insbesondere zeitlich verändern kann, beleuchtet. In Schritt 31 werden dann entsprechend Bilder von dem beleuchteten Auge aufgenommen.

In Schritt 32 wird die Augenrefraktion basierend auf den Bildern bestimmt. Dies kann grundsätzlich wie in den in der Einleitung erwähnten Vorrichtungen, welche eine Beleuchtung aus verschiedenen Richtungen benutzen, geschehen, wobei bei dem Ausführungsbeispiel der Fig. 3 die Anzeige des Smartphones als Beleuchtung dient.

Die so bestimmte Augenrefraktion kann auf verschiedene Weise verwendet werden, wie beispielhaft mit Schritt 33 bis 36 der Fig. 3 angedeutet. Die Schritte 33 bis 36 können unabhängig voneinander implementiert sein und können bei anderen Ausführungsbeispielen auch ganz oder teilweise weggelassen sein.

Beispielsweise wird bei 33 die bestimmte Augenrefraktion verwendet, um eine Sehhilfe (beispielsweise Brillen oder Kontaktlinsen) entsprechend anzupassen. Bei 34 wird die bestimmte Refraktion als Grundlage für eine Therapie verwendet. Bei 35 erfolgt eine Datenübermittlung, beispielsweise zu medizinischem Fachpersonal wie einem Arzt oder einem Optiker. Beispielsweise können, wenn die Refraktion bestimmt würde, entsprechende Daten an einen Optiker übermittelt werden, welcher dann eine entsprechende Brille bereitstellen kann. Eine Datenübermittlung ist beispielsweise auch direkt an einem Brillenhersteller oder Brillenglashersteller möglich, welcher dann entsprechende Brillen oder Brillengläser bereitstellen kann. Vor einer derartigen Bestellung kann eine entsprechende Rückfrage an einen Benutzer und/oder medizinisches Fachperson erfolgen. Schließlich kann die bestimmte Augenrefraktion auch einfach bei 36 angezeigt werden, oder es kann basierend auf der Augenrefraktion auf einer Anzeige eine Empfehlung ausgesprochen werden, beispielsweise eine Empfehlung, einen Augenarzt aufzusuchen.

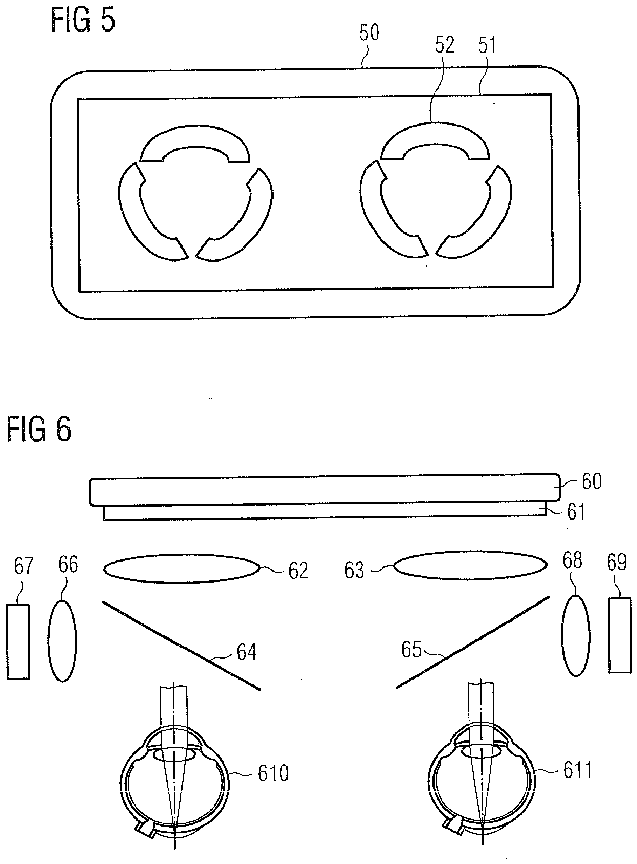

Wie bereits erläutert, kann zur Bestimmung der Refraktion ein Auge (oder beide Augen) eines Benutzers, d.h. einer zu untersuchenden Person, strukturiert beleuchtet werden. Insbesondere kann eine Beleuchtung sequenziell aus verschiedenen Richtungen erfolgen, ähnlich dem eingangs diskutierten Stand der Technik, wobei bei Ausführungsbeispielen der Erfindung im Gegensatz zu dem diskutierten Stand der Technik die Beleuchtung mithilfe einer Anzeige einer mobilen Computervorrichtung, wie beispielsweise einem Smartphone, und nicht mittels diskreter Lichtquellen wie Leuchtdioden erfolgt. Beispiele einer derartigen Beleuchtung werden nunmehr unter Bezugnahme auf die Fig. 4 und 5 näher erläutert.

Als erstes Beispiel ist in Fig. 4 eine mobile Computervorrichtung 40, beispielsweise ein Smartphone oder ein Tablet-Computer, mit einer Anzeige (Display) 41 dargestellt. In Fig. 4 können auf der Anzeige 41 getrennt für linkes und rechtes Auge Lichtquellenpunkte 42, welche jeweils eine einstellbare Ringbeleuchtung bilden, um das jeweilige Auge aus verschiedenen Richtungen zu beleuchten, dargestellt werden. Dabei könnten die einzelnen Lichtquellenpunkte 42 beispielsweise sequenziell oder auch in Gruppen aktiviert werden, um so je nach aktiviertem Lichtquellenpunkt 42 das Auge aus einer jeweiligen Richtung zu beleuchten. Der Leuchtdichtepegel der Lichtquellenpunkte 42 kann dabei einstellbar sein, d.h. die Helligkeit der Lichtquellenpunkte 42 kann je nach Bedarf innerhalb der Möglichkeiten, die die Anzeige 41 bietet, veränderbar sein. Auch die Farbe kann einstellbar sein.

Zudem ist auch eine Umfeldleuchtdichte L_u einstellbar, d.h. der Hintergrund (der Teil der Anzeige 41, welcher nicht dazu dient, eine gerade aktive Punktwelle 42 darzustellen) kann ebenso einstellbar sein. Die Lichtquellenpunkte 42 in Fig. 4 sind dabei z.B. Kreise einer bestimmten Größe, also keine Punkte im mathematischen Sinn, und können näherungsweise als Punktlichtquellen dienen.

Die Beleuchtung mit Lichtquellenpunkten der Fig. 4 dient lediglich als Beispiel, und es sind auch andere Beleuchtungen möglich. So zeigt Fig. 5 eine weitere Möglichkeit. Hier werden auf einer Anzeige 51 einer mobilen Computervorrichtung 50 Ringsegmente 52 angezeigt, welche wahlweise einzeln oder in Gruppen aktivierbar und deaktivierbar sind. Wie bei dem Ausführungsbeispiel der Fig. 4 können auch bei Fig. 5 sowohl eine Leuchtdichte der Kreissegmente 52 als auch eine Hintergrundleuchtdichte (Umfeldleuchtdichte) einstellbar sein.

Auch andere Formen sind möglich. Beispielsweise können konzentrische Ringe verschiedener Größen ebenso als strukturierte Beleuchtung dienen, oder einzelne Punkte, welche sich auch auf der Anzeige bewegen können. In den Fig. 4 und 5 sind jeweils Punktwellen 42 und Ringsegmente 52 für linkes und rechtes Auge dargestellt. In diesem Fall ist die Anzeige wie unter Bezugnahme auf Fig. 2 erläutert geteilt. Bei anderen Ausführungsbeispielen kann die Anzeige auch nur zur Darstellung einer Beleuchtung für ein Auge verwendet werden. In diesem Fall kann beispielsweise die Position der mobilen Computervorrichtung 50 je nach zu untersuchendem Auge verändert werden.

Durch Veränderung der Leuchtdichten wird erfindungsgemäß skotopisches Sehen (auch als Nachtsehen oder Stäbchensehen bezeichnet), photopisches Sehen (auch als Tagsehen oder Zapfensehen bezeichnet) sowie der Übergangsbereich hierzwischen (mesopisches Sehen oder Dämmerungssehen) getrennt untersucht werden.

Als nächstes werden verschiedene Systeme gemäß Ausführungsbeispielen diskutiert, bei welchen mittels einer erfindungsgemäßen mobilen Computervorrichtung wie einem Smartphone, eine objektive Refraktionsbestimmung durchführbar ist. Verschiedene Varianten dieser Systeme werden unter Bezugnahme auf die Fig. 6 bis 12 erläutert. Um Wiederholungen zu vermeiden, sind dabei gleiche oder einander entsprechenden Elemente mit den gleichen Bezugszeichen gekennzeichnet und werden nicht wiederholt erläutert.

Ein erstes Ausführungsbeispiel ist in Fig. 6 dargestellt. Dabei umfasst das System der Fig. 6 eine mobile Computervorrichtung 60, z.B. ein Smartphone oder Tablet, mit einer Anzeige 61, welches zum Darstellen einer strukturierten Beleuchtung für ein linkes Auge 610 und ein rechtes Auge 611 eines Benutzers dient. Die mobile Computervorrichtung 60 kann dabei wie unter Bezugnahme auf die Fig. 1 und 2 diskutiert ausgestaltet sein, die unter Bezugnahme auf Fig. 3 diskutierten Funktionen ausführen und/oder die unter Bezugnahme auf die Fig. 4 und 5 diskutierte Beleuchtung bereitstellen.

In Fig. 6 ist die mobile Computervorrichtung 60 beispielsweise in einer Halterung bereitgestellt, in welcher auch die übrigen in Fig. 6 dargestellten Komponenten bereitgestellt sind bzw. mit dieser gekoppelt sind. Eine derartige Halterung kann beispielsweise am Kopf getragen werden (beispielsweise als sogenanntes Head-Mounted Display, HMD).

Bei dem Ausführungsbeispiel der Fig. 6 umfasst das System weiter Optiken 62, 63, um ein auf der Anzeige 61 dargestelltes Bild zu dem linken Auge 610 bzw. dem rechten Auge 611 abzubilden. Die Optiken 62, 63 können, wie oben diskutiert, beispielsweise in einer Betrachtungsvorrichtung wie einem Head-Mounted Display bereitgestellt sein. Während die Optiken 62, 63 zur Vereinfachung in Fig. 6 als einzelne Linsen dargestellt sind, können die Optiken 62, 63 auch jeweils mehrere optische Elemente, beispielsweise Linsen, umfassen.

Zur Aufnahme eines Bildes des linken Auges 610 ist eine erste Kameraeinrichtung mit einem Bildsensor 67, beispielsweise ein CMOS-Sensor oder ein CCD-Sensor, und mit einer Kameraoptik 66 bereitgestellt. Zur Aufnahme eines Bildes des rechten Auges ist in entsprechender Weise eine zweite Kameraeinrichtung mit einem Bildsensor 69 und mit einer Kameraoptik 68 bereitgestellt. Auch die Kameraoptiken 66, 68 können eine oder mehrere Linsen oder andere optische Elemente umfassen.

Zur Bildaufnahme weist die Vorrichtung der Fig. 6 Strahlteiler 64, 65 auf. Mittels des Strahlteilers 64, 65 wird das Licht von den Augen 610, 611 zu den Bildsensoren 67 bzw. 69 gelenkt. Licht von der Anzeige 61 gelangt durch die Strahlteiler 64, 65 hindurch zu den Augen 610, 611. Die Strahlteiler 64, 65 können beispielsweise teildurchlässige Spiegel sein.

Die ersten und zweiten Kameraeinrichtungen können bei manchen Ausführungsbeispielen mit der mobilen Computervorrichtung 60 gekoppelt sein, beispielsweise drahtlos (beispielsweise über Bluetooth) oder auch drahtgebunden (beispielsweise über einen USB-Anschluss oder eine andere Verbindung). Bei wieder anderen Ausführungsbeispielen können sowohl die ersten und zweiten Kameraeinrichtungen als auch die mobile Computervorrichtung 60 von einer zusätzlichen (nicht dargestellten) Vorrichtung gesteuert werden.

Durch das Bereitstellen zweier Kameraeinrichtungen 66-69 kann neben der objektiven Refraktionsbestimmung für die Augen 610, 611 auch eine Untersuchung des stereoskopischen Sehens (beide Augen gemeinsam) durchgeführt werden.

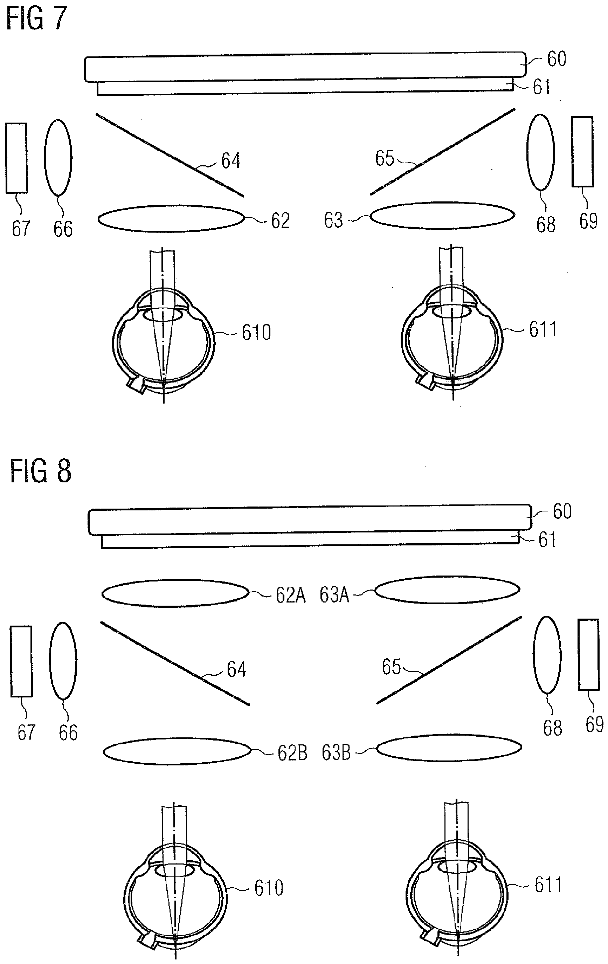

Als nächstes werden Variationen des Systems unter Bezugnahme auf die Fig. 7 bis 12 beschrieben. Dabei werden, wie bereits erläutert, im Wesentlichen die Unterschiede zur Fig. 6 oder zu anderen vorher beschriebenen Figuren diskutiert, und Elemente, welche gleich bleiben, werden nicht nochmals diskutiert.

In Fig. 7 ist verglichen mit Fig. 6 die Position der Strahlteiler 64, 65 mit der Position der Optiken 62, 63 vertauscht. In Fig. 8 sind die Optiken 62A, 62B für die Optik 62 und in Optiken 63A, 63B für die Optik 63 zweigeteilt. Der Strahlteiler 64 ist zwischen den Optiken 62A und 62B angeordnet, und der Strahlenteiler 65 ist zwischen den Optiken 63A und 63B angeordnet. Bei manchen Ausführungsbeispielen können die Strahlteiler 64 und 65 auch innerhalb von Linsen der Optiken 62, 63 angeordnet sein. In anderen Worten zeigen die Fig. 6 bis 8, dass die Anordnung der Strahlteiler 64, 65 und der Optiken 62, 63 variabel ist.

Zu bemerken ist, dass die Optiken 62B, 63B auch austauschbar sein können, was, wie später erläutert werden wird, eine subjektive Refraktionsbestimmung ermöglichen kann.

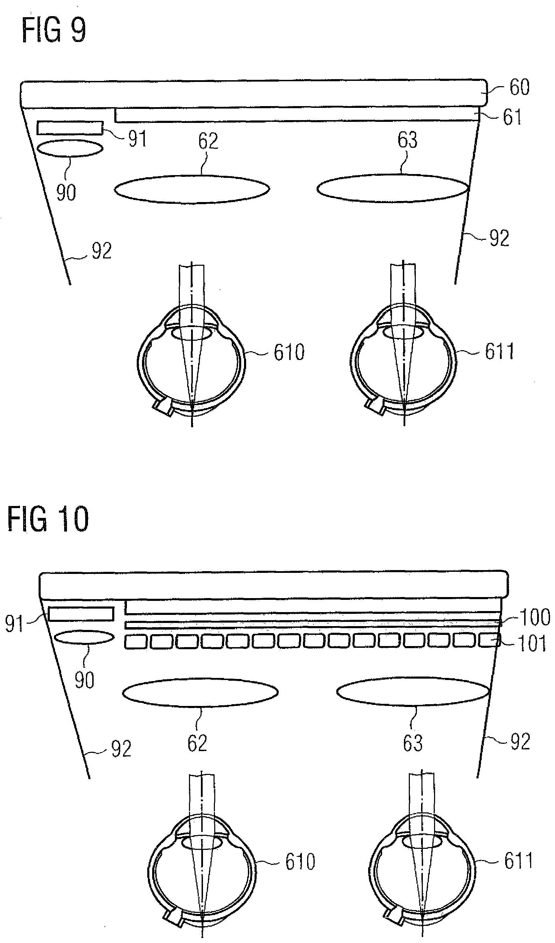

Bei den unter Bezugnahme auf die Figuren 6-8 diskutierten Ausführungsbeispiele werden zu der mobilen Computervorrichtung 60 externe Kameraeinrichtungen (66-69) verwendet, um ein Bild der Augen 610, 611 aufzunehmen. Bei anderen Ausführungsbeispielen kann hierfür eine interne Kamera der mobilen Computervorrichtung 60 verwendet werden. Ein derartiges Ausführungsbeispiel ist in Fig. 9 dargestellt.

Bei dem Ausführungsbeispiel der Fig. 9 weist die mobile Computervorrichtung 60 eine Kameraeinrichtung mit einem Bildsensor 91 und einer Kameraoptik 90 auf. Der Bildsensor 91 kann beispielsweise ein CMOS-Sensor oder ein CCD-Sensor sein. Bei dem dargestellten Ausführungsbeispiel erfolgt wiederum über die Anzeige 61 der mobilen Computervorrichtung 60 eine strukturierte Beleuchtung der Augen, und ein oder beide Augen werden über den Bildsensor 91 aufgenommen, um somit eine objektive Refraktionsbestimmung durchführen zu können. Mit 92 ist ein Gehäuse oder dergleichen bezeichnet, welches insbesondere das Eindringen von Streulicht verhindern oder verringern kann.

Je nach Ausgestaltung der Kameraeinrichtung 90, 91 kann es sein, dass nur ein Auge (in Fig. 9 beispielsweise das Auge 610) aufgenommen werden kann. In diesem Fall muss beispielsweise zur Refraktionsbestimmung des Auges 611 die Ausrichtung der mobilen Computervorrichtung 60 umgedreht werden. Bei anderen Ausführungsbeispielen können zusätzliche optische Elemente (beispielsweise Strahlteiler) bereitgestellt sein, um Licht vom Auge 611 zu dem Bildsensor 91 zu lenken. Bei anderen Ausführungsbeispielen kann die mobile Computervorrichtung 60 zwei Kameras aufweisen.

Dabei wird durch Benutzung einer internen Kamera der mobilen Computervorrichtung 60 der Aufbau insofern vereinfacht, als dass keine zusätzliche externe Kameravorrichtung, ggf. verbunden mit optischen Elementen wie Strahlteilern (beispielsweise 64, 65 der Figuren 6-8) nötig sind. Auf der anderen Seite kann je nach Lage der Kamera wie oben erläutert nur ein Auge aufgenommen werden, was die Augenuntersuchung ggf. verlängern kann. Zudem ist es bei einer Kamera, die simultan nur ein Auge aufnehmen kann, nicht möglich, auch stereoskopisches Sehen zu messen.

Bei den Ausführungsbeispielen der Figuren 6-9 werden Betrachtungsoptiken 62, 63 beispielsweise eines Headup-Displays oder einer anderen Betrachtungsvorrichtung verwendet. Derartige Betrachtungsvorrichtungen für Smartphones sind kommerziell erhältlich. Je nach Qualität der Optiken 62, 63 und der verwendeten strukturierten Beleuchtung können unter Umständen die Optiken 62, 63 unzureichend sein, um eine gewünschte Abbildung der auf der Anzeige 61 dargestellten Beleuchtung auf die Augen 610, 611 zu gewährleisten.

In diesem Fall können beispielsweise, wie in Fig. 10 dargestellt, eine zusätzliche Mikrolinsenanordnung 101 vor der Anzeige 61 bereitgestellt werden. Diese Mikrolinsenanordnung kann eine gewünschte Abbildung der auf der Anzeige 61 dargestellten Beleuchtung (beispielsweise Punktlichtquellen 42 der Fig. 2, 4 oder der Ringsegmente 52 der Fig. 5) auf die Augen 610, 611 gewährleisten. Bei einem bevorzugten Ausführungsbeispiel ist die Mikrolinsenanordnung 101 als Mikrolinsenfolie bereitgestellt, welche einfach auf die Anzeige 61 aufgeklebt oder anders aufgebracht werden kann. Auf diese Weise kann die Mikrolinsenanordnung 101 auf einfache Weise einem Endanwender bereitgestellt werden.

Zudem weist bei dem Ausführungsbeispiel der Fig. 10 das dargestellte System einen Filter 100 auf, welcher ebenfalls als Folie bereitgestellt werden kann und zwischen der Anzeige 61 und der Mikrolinsenanordnung 101 angeordnet ist. Auch andere Anordnungen sind möglich. Der Filter 100 kann bei manchen Ausführungsbeispielen auch mit den Mikrolinsen 101 in einer einzigen Folie bereitgestellt sein, welche dann auf die Anzeige 61 geklebt oder anders aufgebracht werden kann. Der Filter 100 kann dabei insbesondere ein Rot- und/oder Infrarotfilter sein, welcher beispielsweise rotes Licht und/oder Infrarotlicht passieren lässt. Die Verwendung von rotem Licht für die Beleuchtung der Augen 610 und 611 kann den Vorteil bieten, dass hierdurch eine geringere Verengung der Augenpupille hervorgerufen wird als beispielsweise mit weißem Licht, da die wahrgenommene Helligkeit geringer ist. Bei anderen Ausführungsbeispielen kann die Farbe der Beleuchtung direkt über die Anzeige 61 eingestellt werden.

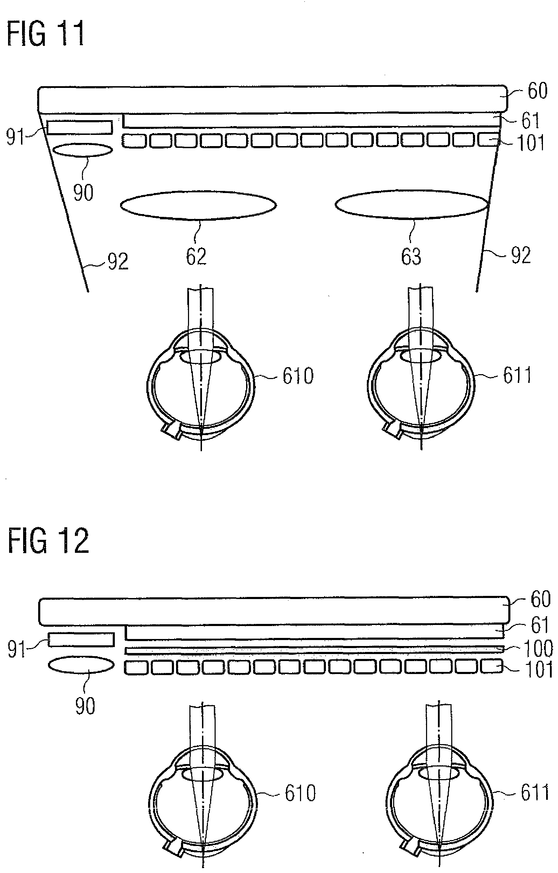

Somit kann der Filter 100 auch weggelassen werden, wie dies im Ausführungsbeispiel der Fig. 11 dargestellt ist. Abgesehen von dem weggelassenen Filter 100 entspricht das Ausführungsbeispiel der Fig. 11 dem Ausführungsbeispiel der Fig. 10.

Bei wieder anderen Ausführungsbeispielen können die Mikrolinsen 101 alleine bereits ausreichend sein, um eine Abbildung einer Beleuchtung auf die Augen 610, 611 zu gewährleisten. Ein derartiges Ausführungsbeispiel ist in Fig. 12 dargestellt.

Bei dem Ausführungsbeispiel der Fig. 12 ist auf der Anzeige 61 lediglich der Filter 100 und die Mikrolinsen 101 bereitgestellt. Die Optik 62, 63 und das Gehäuse 92 sind hingegen weggelassen, d.h., in diesem Fall kann die mobile Computervorrichtung 60 beispielsweise ohne ein Head-Mounted-Display oder dgl. bereitgestellt werden. Dabei kann beispielsweise ein Benutzer angewiesen sein, die mobile Computervorrichtung 60 in einer festgelegten Distanz zu seinen Augen 610, 611 zu halten. Bei manchen Ausführungsbeispielen kann die korrekte Ausrichtung zu den Augen 610, 611 auch über die Kameraeinrichtung 90, 91 kontrolliert werden. Beispielsweise kann mittels eines entsprechenden Moduls wie dem Analysemodul 16 der Fig. 1 überprüft werden, ob die Augen relativ zu dem Bildsensor 91 eine gewünschte Position haben bzw. auf dem Bildsensor 91 in einer gewünschten Größe abgebildet werden. Auch können andere Hilfsmittel, wie ein Maßstab, zur Positionierung bereitgestellt werden. Bei dem Ausführungsbeispiel der Fig. 12 muss einem Benutzer beispielsweise lediglich eine Folie mit dem Filter 100 und den Mikrolinsen 101 zum Aufkleben oder anderweitig Aufbringen auf die Anzeige 61 bereitgestellt werden. Bei anderen Ausführungsbeispielen kann der Filter 100 auch weggelassen sein.

Es ist zu bemerken, dass Filter 100 und/oder Mikrolinsenfolie 101 auch bei den Ausführungsbeispielen der Figuren 6-9 bereitgestellt werden können.

Wie aus den Figuren 6-12 ersichtlich, sind also verschiedene Systeme und Aufbauten möglich, um eine objektive Refraktionsbestimmung mittels einer mobilen Computervorrichtung wie einem Smartphone oder einem Tablet zu erhalten. Dabei können zusätzlich noch andere Messungen, wie beispielsweise allgemein ein Seh-/Visustest, insbesondere auch ein stereoskopischer Test, durchgeführt werden.

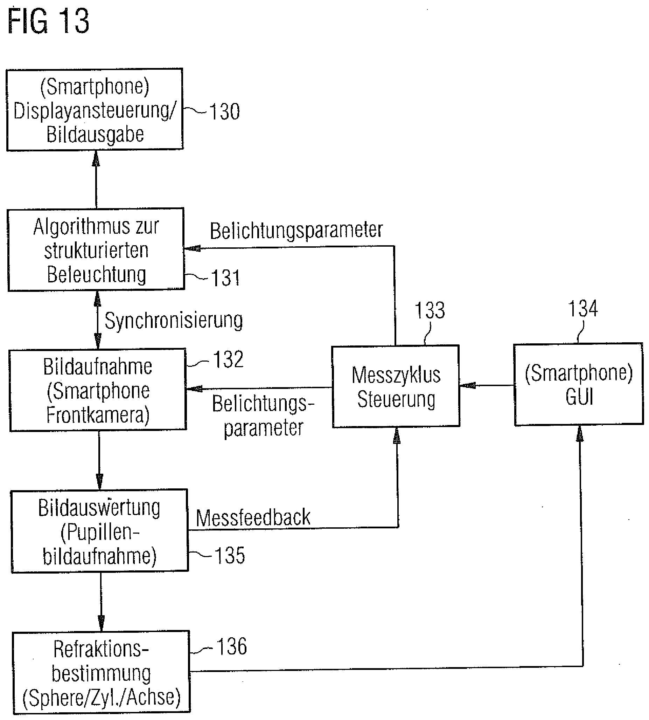

In Fig. 13 ist eine mögliche Funktionsweise derartiger Systeme zur Refraktionsbestimmung nochmals dargestellt. Bei der Darstellung der Fig. 13 wird ein Smartphone als Beispiel für eine mobile Computervorrichtung verwendet, es können jedoch auch andere mobile Computervorrichtungen, beispielsweise Tablet-Computer oder mobile Spielgeräte, verwendet werden. Die verschiedenen unter Bezugnahme auf Fig. 13 im Folgenden erläuterten Funktionalitäten können beispielsweise mittels der vorstehend beschriebenen Vorrichtungen und Systeme implementiert sein, beispielsweise mittels einer entsprechenden Applikation auf einem Smartphone. Somit sind die verschiedenen Blöcke der Fig. 13 nicht notwendigerweise als getrennte Einheiten implementiert, sondern manche der Blöcke können auch gemeinsam mittels einer Applikation auf einer mobilen Computervorrichtung implementiert sein.

Die Bedienung erfolgt dabei wie durch einen Block 134 dargestellt über eine Benutzerschnittstelle der mobilen Computervorrichtung, beispielsweise des Smartphones. Eine derartige Benutzerschnittstelle kann insbesondere eine grafische Benutzerschnittstelle sein, auch als GUI, vom englischen Graphical User Interface, bezeichnet. Hierzu kann insbesondere eine berührungsempfindliche Anzeige (so genannter Touchscreen) verwendet werden. Mittels der Benutzerschnittstelle 134 kann insbesondere eine Messzyklussteuerung 133, welche die Refraktionsbestimmung steuert, gestartet und/oder eingestellt werden.

Die Messzyklussteuerung 133 übergibt Beleuchtungsparameter an einen Algorithmus zur strukturierten Beleuchtung 131, welcher wiederum eine Anzeige, beispielsweise ein Display, der mobilen Computervorrichtung bei 130 ansteuert, um eine gewünschte Beleuchtung zu erzielen. Beispielsweise kann eine Beleuchtung wie unter Bezugnahme auf Fig. 4 oder 5 diskutiert auf diese Weise realisiert werden. Die Beleuchtungsparameter können dabei beispielsweise eine gewünschte Hintergrundhelligkeit, eine gewünschte Art der Beleuchtung (beispielsweise wie in Fig. 4 oder in Fig. 5 dargestellt) und dgl. umfassen. Zudem steuert die Messzyklussteuerung 133 eine Bildaufnahme 132, beispielsweise mittels einer Frontkamera eines Smartphones wie in den Figuren 9-12 dargestellt oder auch mit einer oder mehreren externen Kameras wie in den Figuren 6-8 dargestellt. Dabei kann die Messzyklussteuerung 133 insbesondere Belichtungsparameter, beispielsweise Belichtungszeit, Blende und/oder Isowert, einstellen. Die strukturierte Beleuchtung 131 ist dabei bei dem Ausführungsbeispiel der Fig. 13 mit der Bildaufnahme 132 synchronisiert. Beispielsweise kann für jede Beleuchtungsrichtung (beispielsweise jede Punktlichtquelle 42 der Fig. 4 und/oder jedes Kreissegment 52 der der Fig. 5) ein Bild (oder mehrere Bilder) aufgenommen werden.

Bei 135 erfolgt dann eine Bildauswertung des bei 132 aufgenommenen Bildes. Dabei kann insbesondere ausgewertet werden, ob die Aufnahme einer oder beider Pupillen korrekt erfolgte. Basierend darauf kann der Messzyklussteuerung 133 ein Messfeedback gegeben werden. Basierend auf dem Messfeedback können dann die Beleuchtungsparameter und/oder die Belichtungsparameter angepasst werden. Beispielsweise kann bei einem verwackelten Bild die Belichtungszeit verkürzt werden, oder eine Beleuchtungsstärke kann erhöht werden, wenn ein auszuwertender Pupillenreflex nicht sichtbar ist.

Bei 136 erfolgt dann eine Refraktionsbestimmung auf Basis der aufgenommenen Bilder.

Dabei kann insbesondere der Pupillenrückreflex (optischer Durchgang des Lichtes durch die Augenlinse gefolgt von einer Reflexion an der Retina und einem zweiten Durchgang durch die Augenlinse) ausgewertet werden. Durch Auswertung des Gradientenverlaufes im Reflexbild bei wechselnder Beleuchtungsrichtung kann dann die Fehlsichtigkeit des Auges (Sphäre, Zylinder, Achse) bestimmt werden, da das Verhalten des Rückreflexes von diesen Parametern abhängt.

Dabei können Systeme wie oben beschrieben dadurch, dass die mobile Computervorrichtung relativ nahe an dem zu vermessenden Auge oder den zu vermessenden Augen angeordnet sind, ein großes Gesichtsfeld erreicht werden, d.h. die Anzeige der mobilen Computervorrichtung kann bei Ausführungsbeispielen einen vergleichsweise großen Teil des Gesichtsfeldes, beispielsweise mehr als 50% oder mehr als 70%, ausfüllen. Hierdurch kann die "Hintergrundbeleuchtung" entsprechend einem gewünschten Leuchtdichteadaptionspegel eingestellt werden sowie ein beliebiges Beleuchtungsmuster für die Refraktionsbestimmung eingestellt werden. Somit kann die optische Refraktionsbestimmung mittels Photorefraktion bei unterschiedlichen Pupillengrößen entsprechend den unterschiedlichen Umfeldleuchtdichten durchgeführt werden. Insbesondere bei Systemen, in welchen der Abstand der mobilen Computervorrichtung zu dem Auge oder den Augen klar definiert ist (beispielsweise bei Verwendung eines Headup-Displays) ist im Gegensatz zu herkömmlichen Handheld-Fotorefraktionmetern keine Entfernungsmessung notwendig.

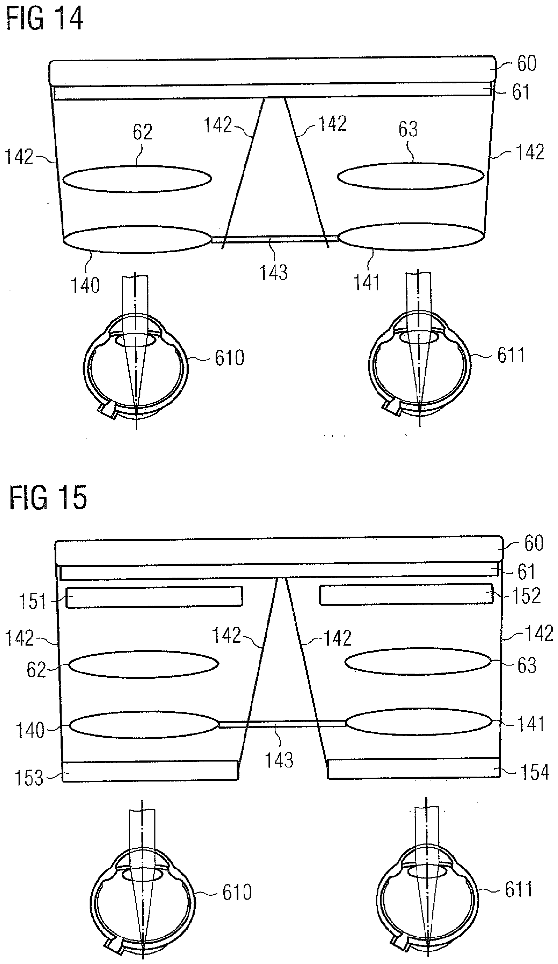

Wie bereits erläutert erfolgt bei den unter Bezugnahme auf die Figuren 3-13 diskutierten Ausführungsbeispielen eine objektive Refraktionsbestimmung durchgeführt. Wie eingangs bereits erläutert können mit anderen Ausführungsbeispielen zusätzlich oder alternativ auch subjektive Refraktionsbestimmungen durchgeführt werden. Derartige Ausführungsbeispiele werden nunmehr unter Bezugnahme auf die Figuren 14-17 erläutert. Elemente der Figuren 14-16, welche Elementen der Figuren 6-12 entsprechen, tragen wiederum die gleichen Bezugszeichen und werden nicht nochmals detailliert erläutert. Insbesondere bezeichnet 60 wiederum eine mobile Computervorrichtung wie ein Smartphone mit einer Anzeige 61, und 610 und 611 bezeichnen zwei Augen einer zu untersuchenden Person. Mit 62 und 63 sind Betrachtungsoptiken einer Betrachtungsvorrichtung wie einem Head-Mounted-Display oder dgl. bezeichnet.

Mit 140 und 141 sind Wechsellinsen oder andere Wechseloptiken bezeichnet, d.h. austauschbare Linsen oder andere Optiken. Mit 143 ist eine Halterung für die austauschbaren Linsen 140, 141, beispielsweise ein Wechselrahmen, bezeichnet. Mit 142 sind Gehäuseteile oder andere Abschattungen bezeichnet, welche zum einen Umgebungslicht abschatten und zum anderen für das linke Auge 610 und das rechte Auge 611 getrennte Lichtkanäle bereitstellen. Zur subjektiven Refraktionsbestimmung werden dann auf der Anzeige 61 Zeichen, Symbole oder Bilder dargestellt, und die zu untersuchende Person gibt an, ob sich bei einem Wechsel der Linsen 140, 141 beispielsweise der Bildeindruck verbessert oder verschlechtert, und/oder ob die Person mehr oder weniger Symbole oder Zeichen erkennen kann.

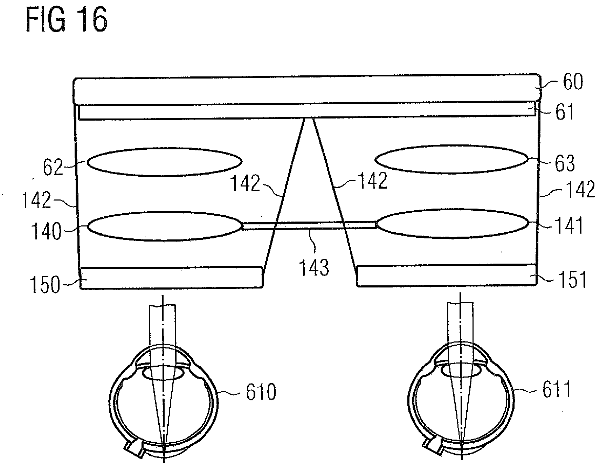

Wie bereits erläutert können bei manchen Ausführungsbeispielen sowohl eine objektive Refraktionsbestimmung als auch eine subjektive Refraktionsbestimmung durchgeführt werden. Bei derartigen kombinierten Systemen können beispielsweise die Linsen 62B und 63B der Fig. 8 den Linsen 140, 141 der Fig. 14 entsprechen. Die subjektive Refraktionsbestimmung kann dabei insbesondere getrennt für linkes Auge 610 und rechtes Auge 611 durchgeführt werden. Es ist jedoch auch eine gemeinsame Benutzung beider Augen (für stereoskopisches Sehen) möglich. In jedem Fall ist es dabei hilfreich, wenn die Lichtwege für linkes Auge 610 und rechtes Auge 611 möglichst gut getrennt sind, damit keine gegenseitige Störung vorliegt. In anderen Worten sollte ein für das linke Auge 610 dargestellter Bildanteil (beispielsweise aus dem Bereich 21A der Fig. 2) möglichst nur zu dem linken Auge 610 gelangen, und ein für das rechte Auge 611 auf der Anzeige 61 dargestellter Bildanteil (beispielsweise aus dem Bereich 21B der Fig. 2) sollte möglichst nur zu dem rechten Auge 611 gelangen. Je nach Design kann dabei die Trennung durch das Gehäuse 142 unzureichend sein. Beispielsweise kann bei manchen Headup-Displays das Gehäuse nicht ganz bis zur Anzeige 61 reichen, beispielsweise um ein leichtes Herausnehmen der Computervorrichtung 60 aus dem Gehäuse zu ermöglichen. In derartigen Fällen können zusätzliche Maßnahmen ergriffen werden. Zwei Möglichkeiten hierzu werden unter Bezugnahme auf die Figuren 15 und 16 erläutert.

Die Systeme der Figuren 15 und 16 beruhen dabei auf dem System der Fig. 14, und gleiche Elemente tragen die gleichen Bezugszeichen und werden nicht nochmals erläutert.

Bei dem Ausführungsbeispiel der Fig. 15 sind Polarisatoren 151 und 152 über der Anzeige 61 angeordnet. Die Polarisatoren 151, 152 können zueinander senkrechte Polarisationen aufweisen (beispielsweise linkszirkular und rechtszirkular oder zwei zueinander senkrechte lineare Polarisationsrichtungen).

Zudem sind an Lichtaustrittsorten des Gehäuses 142 Analysatoren 153, 154 angeordnet. Eine Polarisationsrichtung des Analysators 153 entspricht dabei derjenigen des Polarisators 151. In ähnlicher Weise entspricht eine Polarisation des Analysators 154 einer Polarisation des Polarisators 152. Auf diese Weise kann bei manchen Ausführungsbeispielen eine verbesserte Trennung der Lichtwege für linkes und rechtes Auge erreicht werden.

Eine weitere Möglichkeit ist in Fig. 16 dargestellt. Bei dem Ausführungsbeispiel der Fig. 16 sind Shutter 160 bzw. 161 für das linke Auge 610 bzw. das rechte Auge 611 bereitgestellt. Die Shutter 160, 161 können beispielsweise LCD-Shutter sein. Durch die Shutter 160, 161 kann abwechselnd das linke und das rechte Auge abgedunkelt werden, und es kann gleichzeitig nur für das jeweils nicht abgedunkelte Auge ein Bild auf der Anzeige 61 (beispielsweise in dem entsprechenden Bereich 21A oder 21B der Fig. 2) dargestellt werden. Bei manchen Ausführungsbeispielen kann dabei der Wechsel zwischen linkem Auge und rechtem Auge so schnell erfolgen, dass für beide Augen gleichzeitig ein Bild dargestellt wird. Auch auf diese Weise kann die Trennung der Lichtwege für das linke Auge 610 und das rechte Auge 611 verbessert werden.

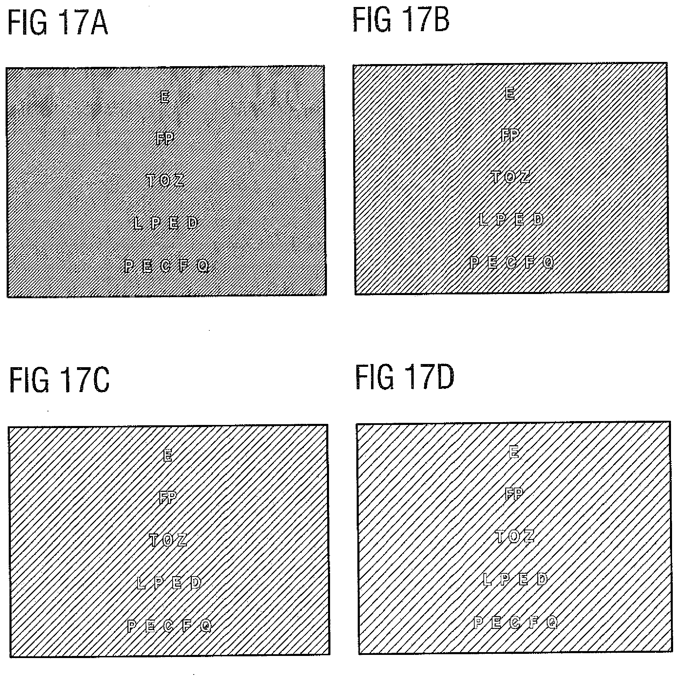

In den Figuren 17A-17D sind verschiedene Beispiele für Buchstaben, welche zur Durchführung einer subjektiven Refraktionsbestimmung angezeigt werden können, dargestellt. Das Größenverhältnis Buchstaben zu Hintergrund ist dabei nicht unbedingt maßstäblich, und bei manchen Ausführungsbeispielen kann der Anteil des Hintergrundes deutlich größer sein im Verhältnis zum Anteil der Buchstaben. Die Größe der Buchstaben kann zudem auch im Gegensatz zu dem Beispiel der Figuren 17A-17B von Zeile zu Zeile variieren. Die zu untersuchende Person kann dann in Abhängigkeit von wechselnden Linsen (beispielsweise den Linsen 140 und 141) angeben, ob die Buchstaben schärfer oder weniger scharf bzw. besser erkennbar oder weniger gut erkennbar sind. Dabei zeigen die Figuren 17A-17D variierende Umfeldleuchtdichten (Lu) und somit variierenden Kontrast zwischen Buchstaben und Hintergrund, womit verschiedene Lichtverhältnisse und verschiedene Arten des Sehens (skotopisch, mesopisch, photopisch) getestet werden können. Dabei wirkt die Umfeldleuchtdichte insbesondere dann, wenn die jeweils dargestellten Bilder einen großen Teil des Gesichtsfeldes (FoV, vom Englischen: Field of View) ausfüllen, als Adapationsleuchtdichten (La), die die Augenadaption bestimmen. Ein derartiges Gesichtsfeld kann z.B. durch eine entsprechende Betrachtungsoptik, z.B. wie vorstehend diskutiert, erreicht werden. Zusätzlich oder alternativ kann eine Helligkeit der dargestellten Buchstaben eingestellt werden, um eine sogenannte Infeldleuchtdichte (Li) einzustellen. So ist der Kontrast zwischen Buchstaben und Hintergrund in der Fig. 17A am größten und in der Fig. 17D am geringsten. Bei anderen Ausführungsbeispielen können zusätzlich oder alternativ zu verschiedenen Kontrasten und Helligkeiten auch verschiedene Farben verwendet werden.

Zu bemerken ist, dass sich das Ergebnis bei der subjektiven Refraktionsbestimmung von dem Ergebnis der objektiven Refraktionsbestimmung unterscheiden kann. Dies liegt daran, dass sich das menschliche Gehirn bis zu einem gewissen Grad an Fehlsichtigkeit gewöhnen kann und diese kompensieren kann. Wenn nun beispielsweise eine Brille verwendet wird, welche eine Fehlsichtigkeit optisch (objektiv) exakt kompensiert, kann das Gehirn dennoch diese Kompensation zunächst weiterführen, was den subjektiven Bildeindruck dann wieder verschlechtert. Bei Ausführungsbeispielen, welche sowohl objektive Refraktionsbestimmung als auch eine subjektive Refraktionsbestimmung ermöglichen, können beispielsweise beide Werte an medizinisches Fachpersonal übermittelt werden, oder es kann beispielsweise für eine Brillen- oder Kontaktlinsenbestimmung ein Kompromiss aus den Ergebnissen der objektiven Refraktionsbestimmung und der subjektiven Refraktionsbestimmung verwendet werden.

Wie bereits erläutert sind die obigen Ausführungsbeispiele nur zur Veranschaulichung gedacht, und sind nicht als einschränkend auszulegen. Varianten und Abwandlungen, welche bezüglich der objektiven Refraktionsbestimmung diskutiert wurden, können ggf. auch bei der subjektiven Refraktionsbestimmung verwendet werden. Beispielsweise kann auch für die subjektive Refraktionsbestimmung eine Mikrolinsenfolie zur Abbildung bereitgestellt werden. Auch andere Variationen sind möglich.

In den Figuren 17A-17D sind verschiedene Beispiele für Buchstaben, welche zur Durchführung einer subjektiven Refraktionsbestimmung angezeigt werden können, dargestellt. Das Größenverhältnis Buchstaben zu Hintergrund ist dabei nicht unbedingt maßstäblich, und bei manchen Ausführungsbeispielen kann der Anteil des Hintergrundes deutlich größer sein im Verhältnis zum Anteil der Buchstaben. Die Größe der Buchstaben kann zudem auch im Gegensatz zu dem Beispiel der Figuren 17A-17B von Zeile zu Zeile variieren. Die zu untersuchende Person kann dann in Abhängigkeit von wechselnden Linsen (beispielsweise den Linsen 140 und 141) angeben, ob die Buchstaben schärfer oder weniger scharf bzw. besser erkennbar oder weniger gut erkennbar sind. Dabei zeigen die Figuren 17A-17D variierende Umfeldleuchtdichten (Lu) und somit variierenden Kontrast zwischen Buchstaben und Hintergrund, womit verschiedene Lichtverhältnisse und verschiedene Arten des Sehens (skotopisch, mesoskopisch, photopisch) getestet werden können. Dabei wirkt die Umfeldleuchtdichte insbesondere dann, wenn die jeweils dargestellten Bilder einen großen Teil des Gesichtsfeldes (FoV, vom Englischen: Field of View) ausfüllen, als Adapationsleuchtdichten (La), die die Augenadaption bestimmen. Ein derartiges Gesichtsfeld kann z.B. durch eine entsprechende Betrachtungsoptik, z.B. wie vorstehend diskutiert, erreicht werden. Zusätzlich oder alternativ kann eine Helligkeit der dargestellten Buchstaben eingestellt werden, um eine sogenannte Infeldleuchtdichte (Li) einzustellen. So ist der Kontrast zwischen Buchstaben und Hintergrund in der Fig. 17A am größten und in der Fig. 17D am geringsten. Bei anderen Ausführungsbeispielen können zusätzlich oder alternativ zu verschiedenen Kontrasten und Helligkeiten auch verschiedene Farben verwendet werden.

Zu bemerken ist, dass sich das Ergebnis bei der subjektiven Refraktionsbestimmung von dem Ergebnis der objektiven Refraktionsbestimmung unterscheiden kann. Dies liegt daran, dass sich das menschliche Gehirn bis zu einem gewissen Grad an Fehlsichtigkeit gewöhnen kann und diese kompensieren kann. Wenn nun beispielsweise eine Brille verwendet wird, welche eine Fehlsichtigkeit optisch (objektiv) exakt kompensiert, kann das Gehirn dennoch diese Kompensation zunächst weiterführen, was den subjektiven Bildeindruck dann wieder verschlechtert. Bei Ausführungsbeispielen, welche sowohl objektive Refraktionsbestimmung als auch eine subjektive Refraktionsbestimmung ermöglichen, können beispielsweise beide Werte an medizinisches Fachpersonal übermittelt werden, oder es kann beispielsweise für eine Brillen- oder Kontaktlinsenbestimmung ein Kompromiss aus den Ergebnissen der objektiven Refraktionsbestimmung und der subjektiven Refraktionsbestimmung verwendet werden.

Bei manchen Ausführungsbeispielen werden Vorrichtungen, Verfahren, Systeme und Computerprogrammprodukte gemäß folgender Absätze bereitgestellt:
Absatz 1. Mobile Computervorrichtung, umfassend:
   eine Anzeige,
   einen Prozessor, und
   einen Speicher mit darin gespeichertem Programmcode ,
   wobei der Programmcode, wenn er auf dem Prozessor ausgeführt wird, bewirkt, dass der Prozessor die Anzeige ansteuert, um auf der Anzeige ein Bild zur Refraktionsbestimmung eines Auges anzuzeigen.
Absatz 2. Mobile Computervorrichtung nach Absatz 1, wobei das Bild auf einem Hintergrund dargestellte Elemente umfasst, wobei eine Helligkeit des Hintergrundes zur Einstellung einer Umfeldleuchtdichte einstellbar ist.
Absatz 3. Mobile Computervorrichtung nach Absatz 2, wobei durch Einstellen der Umfeldleuchtdichte eine Adaptionsleuchtdichte einstellbar ist.
Absatz 4. Mobile Computervorrichtung nach Absatz 2 oder 3, wobei die Helligkeit des Hintergrunds zum wahlweisen Refraktionsbestimmen für mindestens zwei aus der Gruppe umfassend skotopisches Sehen, photopisches Sehen und mesoskopisches Sehen einstellbar ist.
Absatz 5. Mobile Computervorrichtung nach einem der Absätze 2-4, wobei eine Helligkeit der Elemente zur Einstellung einer Infeldleuchtdichte einstellbar ist.
Absatz 6. Mobile Computervorrichtung nach einem der Absätze 1-5, wobei das Bild ein Bild zur subjektiven Refraktionsbestimmung umfasst.
Absatz 7. Mobile Computervorrichtung nach Absatz 6, wobei das Bild Zeichen und/oder Symbole umfasst.
Absatz 8. Mobile Computervorrichtung nach einem der Absätze 1-7, wobei das Bild eine strukturierte Beleuchtung zur Beleuchtung des Auges für eine objektive Refraktionsbestimmung umfasst.
Absatz 9. Mobile Computervorrichtung nach Absatz 8, wobei die strukturierte Beleuchtung zeitlich veränderlich ist, um das Auge aus verschiedenen Richtungen zu beleuchten.
Absatz 10. Mobile Computervorrichtung nach Absatz 8 oder 9, wobei die strukturierte Beleuchtung wahlweise ansteuerbare kreisförmig angeordnete Lichtquellenpunkte oder Ringsegmente umfasst.
Absatz 11. Mobile Computervorrichtung nach einem der Absätze 8-10, weiter umfassend eine Kameraeinrichtung zum Aufnehmen eines Pupillenreflexes in Antwort auf die strukturierte Beleuchtung.
Absatz 12. Mobile Computervorrichtung nach einem der Absätze 1-11, wobei die mobile Computervorrichtung eine Schnittstelle zum Koppeln mit einer externen Kamera und zum Empfangen eines aufgenommenen Pupillenreflexes in Antwort auf die strukturierte Beleuchtung von der externen Kamera umfasst.
Absatz 13. Mobile Computervorrichtung nach Absatz 11 oder 12, wobei der Programmcode, wenn er auf dem Prozessor ausgeführt wird, eine Refraktionsbestimmung des Auges auf Basis des aufgenommenen Pupillenreflexes durchführt.
Absatz 14. System, umfassend:
   eine mobile Computervorrichtung nach einem der Absätze 1-13, und
   eine Betrachtungsoptik zum Betrachten der mobilen Computervorrichtung mit mindestens einem Auge einer zu untersuchenden Person.
Absatz 15. System nach Absatz 14, wobei die Betrachtungsoptik eine erste Optik für ein erstes Auge und eine zweite Optik für ein zweites Auge umfasst.
Absatz 16. System nach Absatz 14 oder 15, wobei die Betrachtungsoptik eine Mikrolinsenanordnung umfasst.
Absatz 17. System nach Absatz 16, wobei die Mikrolinsenanordnung eine auf der Anzeige der mobilen Computervorrichtung anzubringende Mikrolinsenfolie umfasst.
Absatz 18. System nach einem der Absätze 14-17, wobei die Betrachtungsoptik einen Farbfilter umfasst.
Absatz 19. System nach Absatz 18, wobei der Farbfilter einen Rotfilter und/oder Infrarotfilter umfasst.
Absatz 20. System nach einem der Absätze 14-19, weiter umfassend einen Strahlteiler, welcher zwischen der Anzeige der mobilen Computervorrichtung und dem mindestens einen Auge angeordnet ist, wobei der Strahlteiler angeordnet ist, von dem Auge ausgehendes Licht zu einer Kameraeinrichtung zu lenken.
Absatz 21. System nach einem der Absätze 14-20, weiter umfassend eine Wechselhalterung für eine Wechseloptik zur subjektiven Refraktionsbestimmung.
Absatz 22. System nach einem der Absätze 14-21, weiter umfassend, für mindestens ein Auge eine Kombination aus Polarisator und Analysator.
Absatz 23. System nach einem der Absätze 14-22, weiter umfassend, für mindestens ein Auge, einen Shutter.
Absatz 24. Computerprogrammprodukt mit einem Programmcode, wobei der Programmcode, wenn er auf einem Prozessor ausgeführt wird, bewirkt, dass der Prozessor eine Anzeige ansteuert, um auf der Anzeige ein Bild zur Refraktionsbestimmung eines Auges anzuzeigen.
Absatz 25. Computerprogrammprodukt nach Absatz 24, wobei das Computerprogrammprodukt zur Implementierung einer mobilen Computervorrichtung nach einem der Absätze 1-13 ausgestaltet ist.

Wie bereits erläutert sind die obigen Ausführungsbeispiele nur zur Veranschaulichung gedacht, und sind nicht als einschränkend auszulegen. Varianten und Abwandlungen, welche bezüglich der objektiven Refraktionsbestimmung diskutiert wurden, können ggf. auch bei der subjektiven Refraktionsbestimmung verwendet werden. Beispielsweise kann auch für die subjektive Refraktionsbestimmung eine Mikrolinsenfolie zur Abbildung bereitgestellt werden. Auch andere Variationen sind möglich.

## Patentansprüche

1. Mobile Computervorrichtung (10; 20; 60), umfassend:
eine Anzeige (11; 21; 61),
einen Prozessor (13), und
einen Speicher mit darin gespeichertem Programmcode (16),
wobei der Programmcode (16), wenn er auf dem Prozessor (13) ausgeführt wird, bewirkt, dass der Prozessor (13) die Anzeige (11) ansteuert, um auf der Anzeige (11) ein Bild zur Refraktionsbestimmung eines Auges (610, 611) anzuzeigen, wobei das Bild auf einem Hintergrund dargestellte Elemente umfasst, wobei eine Helligkeit des Hintergrundes zur Einstellung einer Umfeldleuchtdichte einstellbar ist,
**dadurch gekennzeichnet, dass** die Helligkeit des Hintergrundes unabhängig von der Helligkeit der Elemente einstellbar ist und dass die Helligkeit des Hintergrundes wahlweise auf einen von mindestens zwei unterschiedlichen vorgegebenen Helligkeitswerten einstellbar ist, wobei die mindestens zwei unterschiedlichen vorgegebenen Helligkeitswerte aus einer Gruppe sind bestehend aus einem ersten vorgegebenen Helligkeitswert in einem Bereich des skotopischen Sehens mit einer ersten Leuchtdichte kleiner als 0,03 cd/m², einem zweiten vorgegebenen Helligkeitswert in einem Bereich des photopischen Sehens mit einer zweiten Leuchtdichte größer als 3 cd/m² und einem dritten Helligkeitswert in einem Bereich des mesopischen Sehens mit einer dritten Leuchtdichte zwischen der ersten Leuchtdichte und der zweiten Leuchtdichte.

2. Mobile Computervorrichtung (10; 20; 60) nach Anspruch 1, **dadurch gekennzeichnet, dass** eine Helligkeit der Elemente zur Einstellung einer Infeldleuchtdichte einstellbar ist.

3. Mobile Computervorrichtung (10; 20; 60) nach einem der vorangegangenen Ansprüche, **dadurch gekennzeichnet, dass** das Bild eine strukturierte Beleuchtung zur Beleuchtung des Auges (610, 611) für eine objektive Refraktionsbestimmung umfasst.

4. Mobile Computervorrichtung (10; 20; 60) nach Anspruch 3, weiter umfassend eine Kameraeinrichtung (90, 91) zum Aufnehmen eines Pupillenreflexes in Antwort auf die strukturierte Beleuchtung.

5. Mobile Computervorrichtung (10; 20; 60) nach einem der Ansprüche 3 oder 4, **dadurch gekennzeichnet, dass** eine Schnittstelle (14) zum Koppeln mit einer externen Kamera (40) und zum Empfangen eines aufgenommenen Pupillenreflexes in Antwort auf die strukturierte Beleuchtung von der externen Kamera (40) vorhanden ist.

6. Mobile Computervorrichtung (10; 20; 60) nach Anspruch 4 oder 5, **dadurch gekennzeichnet, dass** der Programmcode (16), wenn er auf dem Prozessor (13) ausgeführt wird, eine Refraktionsbestimmung des Auges (610, 611) auf Basis des aufgenommenen Pupillenreflexes durchführt.

7. Mobile Computervorrichtung (10; 20; 60) nach einem der Ansprüche 3 bis 6, **dadurch gekennzeichnet, dass** die strukturierte Beleuchtung zeitlich veränderlich ist, um das Auge (610, 611) aus verschiedenen Richtungen zu beleuchten.

8. Mobile Computervorrichtung (10; 20; 60) nach einem der Ansprüche 3 bis 7, **dadurch gekennzeichnet, dass** die strukturierte Beleuchtung wahlweise ansteuerbare kreisförmig angeordnete Lichtquellenpunkte (42) oder Ringsegmente (52) umfasst.

9. System, umfassend:
eine mobile Computervorrichtung nach einem der Ansprüche 1 bis 8, und
eine Betrachtungsoptik (62, 63, 100, 101) zum Betrachten der mobilen Computervorrichtung mit mindestens einem Auge (610, 611) einer zu untersuchenden Person, wobei die Betrachtungsoptik (62, 63, 100, 101) eine erste Optik (62) für ein erstes Auge und eine zweite Optik (63) für ein zweites Auge umfasst.

10. Verfahren zur Augenuntersuchung, umfassend
Anzeigen eines Bildes zur Refraktionsbestimmung eines Auges (610, 611) auf einer Anzeige (11) einer mobilen Computervorrichtung (10; 20; 60), wobei das Bild auf einem Hintergrund dargestellte Elemente umfasst, und
Einstellen einer Helligkeit des Hintergrundes zur Einstellung einer Umfeldleuchtdichte **dadurch gekennzeichnet, dass** die Helligkeit des Hintergrundes unabhängig von der Helligkeit der Elemente eingestellt wird, und dass die Helligkeit des Hintergrundes wahlweise zur Refraktionsbestimmung für mindestens zwei Arten von Sehen aus der Gruppe bestehend aus skotopisches Sehen mit einer ersten Leuchtdichte kleiner als 0,03 cd/m², photopisches Sehen mit einer zweiten Leuchtdichte größer als 3 cd/m² und mesopisches Sehen mit einer dritten Leuchtdichte zwischen der ersten Leuchtdichte und der zweiten Leuchtdichte eingestellt wird.

11. Verfahren nach Anspruch 10, weiter umfassend:
Einstellen einer Helligkeit der Elemente zur Einstellung einer Infeldleuchtdichte.

12. Verfahren nach einem der Ansprüche 10 oder 11, wobei das Bild eine strukturierte Beleuchtung zur Beleuchtung des Auges (610, 611) für eine objektive Refraktionsbestimmung umfasst.

13. Verfahren nach Anspruch 12, weiter umfassend:
Aufnehmen eines Pupillenreflexes in Antwort auf die strukturierte Beleuchtung, und
Durchführen einer Refraktionsbestimmung des Auges (610, 611) auf Basis des aufgenommenen Pupillenreflexes mittels eines auf einem Prozessor (13) der mobilen Computervorrichtung (10; 20; 60) ausgeführten Programmcodes.

14. Verfahren nach einem der Ansprüche 12 oder 13, weiter umfassend:
zeitliches Verändern der strukturierten Beleuchtung, um das Auge (610, 611) aus verschiedenen Richtungen zu beleuchten.

15. Computerprogrammprodukt, welches in einen Speicher einer mobilen Computervorrichtung (10; 20; 60) ladbar ist, umfassend Softwarecode, welcher das Verfahren nach einem der Ansprüche 10-14 ausführt, wenn er auf der mobilen Computervorrichtung (10; 20; 60) ausgeführt wird.

## Claims

1. Mobile computer device (10; 20; 60), comprising:
a display (11; 21; 61),
a processor (13), and
a memory with program code (16) stored therein, wherein the program code (16), when it is executed on the processor (13), has the effect that the processor (13) drives the display (11) to display an image for determining the refraction of an eye (610, 611) on the display (11), wherein the image comprises elements represented on a background, wherein a brightness of the background is settable for setting a surround luminance,
**characterized in that** the brightness of the background is settable independently of the brightness of the elements and **in that** the brightness of the background is optionally settable to one of at least two different predefined brightness values, wherein the at least two different predefined brightness values are from a group consisting of a first predefined brightness value in a range of scotopic vision with a first luminance of less than 0.03 cd/m², a second predefined brightness value in a range of phototopic vision with a second luminance of greater than 3 cd/m² and a third brightness value in a range of mesopic vision with a third luminance between the first luminance and the second luminance.

2. Mobile computer device (10; 20; 60) according to Claim 1, **characterized in that** a brightness of the elements is settable for setting an infield luminance.

3. Mobile computer device (10; 20; 60) according to either of the preceding claims, **characterized in that** the image comprises a structured illumination for illuminating the eye (610, 611) for an objective refraction determination.

4. Mobile computer device (10; 20; 60) according to Claim 3, further comprising a camera unit (90, 91) for recording a pupillary light reflex in response to the structured illumination.

5. Mobile computer device (10; 20; 60) according to either of Claims 3 and 4, **characterized in that** an interface (14) for coupling to an external camera (40) and for receiving a recorded pupillary light reflex in response to the structured illumination from the external camera (40) is present.

6. Mobile computer device (10; 20; 60) according to Claim 4 or 5, **characterized in that** the program code (16), when it is executed on the processor (13), carries out a determination of the refraction of the eye (610, 611) on the basis of the recorded pupillary light reflex.

7. Mobile computer device (10; 20; 60) according to any of Claims 3 to 6, **characterized in that** the structured illumination is temporally variable in order to illuminate the eye (610, 611) from different directions.

8. Mobile computer device (10; 20; 60) according to any of Claims 3 to 7, **characterized in that** the structured illumination comprises optionally drivable, circularly arranged light source points (42) or ring segments (52).

9. System, comprising:
a mobile computer device according to any of Claims 1 to 8, and
a viewing optical unit (62, 63, 100, 101) for viewing the mobile computer device with at least one eye (610, 611) of a person to be examined, wherein the viewing optical unit (62, 63, 100, 101) comprises a first optical unit (62) for a first eye and a second optical unit (63) for a second eye.

10. Method for eye examination, comprising
displaying an image for determining the refraction of an eye (610, 611) on a display (11) of a mobile computer device (10; 20; 60), wherein the image comprises elements represented on a background, and
setting a brightness of the background for setting a surround luminance,
**characterized in that** the brightness of the background is set independently of the brightness of the elements and **in that** the brightness of the background is optionally set for the refraction determination for at least two types of vision from the group consisting of scotopic vision with a first luminance of less than 0.03 cd/m², photopic vision with a second luminance of greater than 3 cd/m² and mesopic vision with a third luminance between the first luminance and the second luminance.

11. Method according to Claim 10, further comprising:
setting a brightness of the elements for setting an infield luminance.

12. Method according to either of Claims 10 and 11, wherein the image comprises a structured illumination for illuminating the eye (610, 611) for an objective refraction determination.

13. Method according to Claim 12, further comprising:
recording a pupillary light reflex in response to the structured illumination, and
carrying out a determination of the refraction of the eye (610, 611) on the basis of the recorded pupillary light reflex by means of a program code executed on a processor (13) of the mobile computer device (10; 20; 60).

14. Method according to either of Claims 12 and 13, further comprising:
temporally varying the structured illumination in order to illuminate the eye (610, 611) from different directions.

15. Computer program product which is loadable into a memory of a mobile computer device (10; 20; 60), comprising software code which performs the method according to any of Claims 10-14 when it is executed on the mobile computer device (10; 20; 60).

## Revendications

1. Dispositif informatique mobile (10 ; 20 ; 60), comprenant :
un afficheur (11 ; 21 ; 61),
un processeur (13), et
une mémoire dans laquelle est stocké un code de programme (16),
dans lequel le code de programme (16), lorsqu'il est exécuté sur le processeur (13), fait en sorte que le processeur (13) commande l'afficheur (11) afin d'afficher sur l'afficheur (11) une image permettant de déterminer la réfraction d'un oeil (610, 611), dans lequel l'image comprend des éléments représentés sur un arrière-plan, dans lequel une luminosité de l'arrière-plan est réglable afin de régler une densité lumineuse ambiante,
**caractérisé en ce que** la luminosité de l'arrière-plan est réglable indépendamment de la luminosité des éléments et **en ce que** la luminosité de l'arrière-plan est réglable de manière sélective sur l'une d'au moins deux valeurs de luminosité prédéterminées différentes, dans lequel les au moins deux valeurs de luminosité prédéterminées différentes appartiennent à un groupe constitué d'une première valeur de luminosité prédéterminée se situant dans un domaine de la vision scotopique ayant une première densité lumineuse inférieure à 0,03 cd/m², d'une deuxième valeur de luminosité prédéterminée se situant dans un domaine de la vision photopique ayant une deuxième densité lumineuse supérieure à 3 cd/m², et d'une troisième valeur lumineuse se situant dans un domaine de la vision mésopique ayant une troisième densité lumineuse comprise entre la première densité lumineuse et la deuxième densité lumineuse.

2. Dispositif informatique mobile (10 ; 20 ; 60) selon la revendication 1, **caractérisé en ce qu'**une luminosité des éléments est réglable afin de régler une densité lumineuse dans le champ.

3. Dispositif informatique mobile (10 ; 20 ; 60) selon l'une quelconque des revendications précédentes, **caractérisé en ce que** l'image comprend un éclairage structuré permettant d'éclairer l'oeil (610, 611) à des fins de détermination objective de la réfraction.

4. Dispositif informatique mobile (10 ; 20 ; 60) selon la revendication 3, comprenant en outre un dispositif à caméra (90, 91) permettant d'acquérir un réflexe de pupille en réponse à l'éclairage structuré.

5. Dispositif informatique mobile (10 ; 20 ; 60) selon l'une quelconque des revendications 3 ou 4, **caractérisé en ce qu'**il est prévu une interface (14) pouvant être couplée à une caméra externe (40) et destinée à recevoir un réflexe de pupille acquis en réponse à l'éclairage structuré par la caméra externe (40).

6. Dispositif informatique mobile (10 ; 20 ; 60) selon la revendication 4 ou 5, **caractérisé en ce que** le code de programme (16), lorsqu'il est exécuté sur le processeur (13), effectue une détermination de la réfraction de l'oeil (610, 611) sur la base du réflexe de pupille acquis.

7. Dispositif informatique mobile (10 ; 20 ; 60) selon l'une quelconque des revendications 3 à 6, **caractérisé en ce que** l'éclairage structuré peut être modifié dans le temps afin d'éclairer l'oeil (610, 611) dans des directions différentes.

8. Dispositif informatique mobile (10 ; 20 ; 60) selon l'une quelconque des revendications 3 à 7, **caractérisé en ce que** l'éclairage structuré comprend des sources lumineuses ponctuelles (42) ou des éléments annulaires (52) pouvant être commandés de manière sélective et agencés de manière circulaire.

9. Système, comprenant :
un dispositif informatique mobile selon l'une quelconque des revendications 1 à 8, et
une optique d'observation (62, 63, 100, 101) destinée à l'observation du dispositif informatique mobile au moyen d'au moins un oeil (610, 611) d'une personne à examiner, dans lequel l'optique d'observation (62, 63, 100, 101) comprend une première optique (62) destinée à un premier oeil et une deuxième optique (63) destinée à un deuxième oeil.

10. Procédé d'examen oculaire, consistant à :
afficher une image à des fins de détermination de la réfraction d'un oeil (610, 611) sur un afficheur (11) d'un dispositif informatique mobile (10 ; 20 ; 60), dans lequel l'image comprend des éléments représentés sur un arrière-plan, et
régler une luminosité de l'arrière-plan afin de régler une densité lumineuse ambiante, **caractérisé en ce que** la luminosité de l'arrière-plan est réglée indépendamment de la luminosité des éléments, et **en ce que** la luminosité de l'arrière-plan est réglée de manière sélective à des fins de détermination de la réfraction pour au moins deux types de vision appartenant au groupe constitué de la vision scotopique ayant une première densité lumineuse inférieure à 0,03 cd/m², une vision photopique ayant une deuxième densité lumineuse supérieure à 3 cd/m², et une vision mésopique ayant une troisième densité lumineuse comprise entre la première densité lumineuse et la deuxième densité lumineuse.

11. Procédé selon la revendication 10, consistant en outre à :
régler une luminosité des éléments afin de régler une densité lumineuse dans le champ.

12. Procédé selon l'une quelconque des revendications 10 ou 11, dans lequel l'image comprend un éclairage structuré permettant d'éclairer l'oeil (610, 611) en vue d'une détermination objective de la réfraction.

13. Procédé selon la revendication 12, consistant en outre à :
acquérir un réflexe de pupille en réponse à l'éclairage structuré, et
effectuer une détermination de la réfraction de l'oeil (610, 611) sur la base du réflexe de pupille acquis au moyen d'un code de programme exécuté sur un processeur (13) du dispositif informatique mobile (10, 20, 60).

14. Procédé selon l'une quelconque des revendications 12 ou 13, consistant en outre à faire varier dans le temps l'éclairage structuré afin d'éclairer l'oeil (610, 611) dans des directions différentes.

15. Produit de programme d'ordinateur pouvant être chargé dans une mémoire d'un dispositif informatique mobile (10 ; 20 ; 60), comprenant un code logiciel qui met en oeuvre le procédé selon l'une quelconque des revendications 10 à 14 lorsqu'il est exécuté sur le dispositif informatique mobile (10 ; 20 ; 60).
